(19) Europäisches Patentamt
European Patent Office
Office européen des brevets

(11) EP 2 286 222 B1

(12) **EUROPEAN PATENT SPECIFICATION**

(45) Date of publication and mention of the grant of the patent:
**24.02.2016 Bulletin 2016/08**

(21) Application number: **09732268.9**

(22) Date of filing: **08.04.2009**

(51) Int Cl.:
***G01N 33/53*** *(2006.01)* ***C12Q 1/42*** *(2006.01)*

(86) International application number:
**PCT/GB2009/050344**

(87) International publication number:
**WO 2009/127855 (22.10.2009 Gazette 2009/43)**

(54) **USE OF A MONOPHOSPHATE ESTER OF A PHTHALEIN COMPOUND AS A SUBSTRATE FOR TARTRATE-RESISTANT ACID PHOSPHATASE B5 (TRAP 5B)**

VERWENDUNG EINES MONOPHOSPHATESTERS EINER PHTHALEINVERBINDUNG ALS SUBSTRAT FÜR TARTRAT-RESISTENTE SAURE PHOSPHATASE B5 (TRAP 5B)

UTILISATION D'UN ESTER MONOPHOSPHATE D'UN COMPOSE PHTHALEINE COMME SUBSTRAT POUR TRAP 5B

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **15.04.2008 GB 0806801**
**19.08.2008 GB 0815105**

(43) Date of publication of application:
**23.02.2011 Bulletin 2011/08**

(73) Proprietor: **Immunodiagnostic Systems Limited**
**Tyne and Wear NE35 9PD (GB)**

(72) Inventors:
- **HOUSTON, Brian**
**Boldon Tyne and Wear NE35 9PD (GB)**
- **LAURIE, David**
**Boldon Tyne and Wear NE35 9PD (GB)**
- **HALLEEN, Jussi**
**FI-20520 Turku (FI)**

(74) Representative: **Shah, Punita et al**
**HGF Limited**
**4th Floor, Merchant Exchange**
**17-19 Whitworth Street West**
**Manchester M1 5WG (GB)**

(56) References cited:
**EP-A- 1 359 161** **EP-A- 1 598 670**
**WO-A-99/50662** **US-A- 3 331 857**

- **ROY A V ET AL: "Sodium thymolphthalein monophosphate: A new acid phosphatase substrate with greater specificity for the prostatic enzyme in serum" CLINICAL CHEMISTRY 1971, vol. 17, no. 11, 1971, pages 1093-1102, XP002535254 ISSN: 0009-9147**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 286 222 B1

**Description**

FIELD OF THE INVENTION

The present invention relates to the use of a monophosphate ester of a phthalein compound as a substrate for Tartrate-Resistant Acid Phosphatase (TRAP), preferably TRAP 5b. The present invention also relates to obtaining a measure of the amount of TRAP 5b in a sample of a subject, as an indication of the rate of bone resorption, and methods related thereto. Also provided is the use of a monophosphate ester of a phthalein compound in obtaining a measure of TRAP 5b in a sample, and kits for the performance of a method of the invention.

BACKGROUND

Bone consists of two major cell types: the osteoblasts responsible for forming bone and osteoclasts responsible for breaking down (resorbing) bone. Osteoclasts are multinucleated cells which burrow into mineralised bone, breaking it down into its component parts. The resorption process comprises attachment of the osteoclast to an osteon. The osteoclast then induces an infolding of its cell membrane and secretes collagenase and other enzymes important in the resorption process. As the bone is resorbed, high levels of calcium, magnesium, phosphate and products of collagen are released into the extracellular fluid. Normally the osteoblasts and osteoclasts are in balance, so that the same amount of bone is resorbed as that which is formed. During childhood, bone formation exceeds resorption, but as the ageing process occurs, resorption exceeds formation.

Several medical conditions are known to be associated with changes in bone resorption rates, perhaps the most widely occurring being osteoporosis. This particular condition is thought to be the result of increased activity of the osteoclasts, resulting a decrease in bone mass, particularly in post-menopausal women. This causes a weakening of the bones and increased susceptibility to fractures. Fractures in women of this age group can be potentially serious, and treatment of these and other symptoms caused by osteoporosis can be expensive and demanding for the medical profession. Diagnosis of those susceptible to osteoporosis has long been recognised as being important, in order to prevent the potentially severe consequences of the condition. Increasingly, osteoporosis is being successfully treated, for example by estrogen replacement therapy or biphosphonate treatment.

An indicator of osteoporosis or susceptibility thereto is a measurement of bone density in a subject, where low bone density suggests excess resorption of bone and therefore likelihood of osteoporosis or other conditions. As this group of people is recognised as being a commercially important market, especially in the western world, numerous methods and instruments have been developed in order to measure bone density. Of these, those which are specific, rapid, and cheap are favoured.

Tartrate-resistant acid phosphatase (TRAP) belongs to the type 5 class of acid phosphatases according to its electrophoretic mobility. It is the most acidic of the seven acid phosphatases identified, and the only one which is resistant to inhibition by Tartrate. TRAP is a basic glycoprotein containing a spin-coupled iron unit at the active site of the molecule. The binuclear iron unit of TRAP contains two ferric ions, which make the protein purple, and as a result it is also known as purple acid phosphatase. This protein has a molecular weight of 32 kd. When the enzyme is reduced, for example, with β-mercaptoethanol, one of the ferric ions is reduced and the enzyme becomes pink. TRAP can function as a protein tyrosine phosphatase *in vitro* and its amino acid sequence contains regions homologous to those of phosphoprotein phosphatases. However, the natural substrates of TRAP are still unknown.

Several studies have implicated TRAP in the bone resorption process. Disruption of the TRAP gene in mice (Hayman, A. R et al Development 122:3151-3162, 1996) results in disrupted endochondral ossification and mild osteoporosis, suggesting that TRAP is required for normal mineralization of cartilage in developing bones and maintaining integrity and turnover of the adult skeleton by a critical contribution to bone matrix resorption.

In humans, the only normal cells in which TRAP is found are the osteoclasts and activated macrophages. It has been found that TRAP is secreted from the osteoclasts into the blood during bone resorption. The concentration of TRAP in serum has therefore been suggested as a marker of bone resorption, and it has been found that the concentration of TRAP in serum correlates with the bone resorption rate (Kraenzlin M. E et al. Journal of Clinical Endocrinology and Metabolism 71 (2): 442-451, 1990; Cheung. C.K et al. Clin. Chem 41(5):679-686, 1995; Chamberlain P. et al. Clin. Chem. 41(10): 1495-1499, 1995 and Halleen J. et al. Journal of Bone and Mineral Research 11 (10): 1444-1452, 1996).

Two isoforms of TRAP have been identified, known as TRAP 5a and TRAP 5b (Lam W. K. W. et al. (Clin. Chem. 24(7):1105-1108, 1978)). TRAP 5a is active at a lower optimum pH than TRAP 5b, thereby allowing functional assays for each isoform of TRAP. The major difficulty in the measurement of serum or plasma concentrations of TRAP5b for purpose of determining, for example, bone resorption rate, is the presence of the 5a isoform of the enzyme which is produced principally by macrophages and dendritic cells. Both isoforms are the product of the *acp5* gene and differ only in respect to variations in post-translational modification. TRAP5b lacks certain sialic acid residues (on the protein's carbohydrate groups) that are present in TRAP5a. The isoforms also differ in that an approximately 14 residue segment

of the amino acid chain has been proteolytically excised from TRAP5b. Otherwise the core proteins are identical and methods for distinguishing the two forms on a purely immunological basis are not available. Consequently, existing commercial immunoassays for TRAP5b take advantage of biochemical differences between the two forms.

TRAP activity can be determined spectrophotometrically e.g. by feeding p-nitrophenyl phosphate (pNPP) as a substrate in the presence of tartrate, which inhibits most other acid phosphatases. However, this method is not very specific, and therefore more specific methods i.e. immunoassays have been developed for the determination of TRAP. Various assays for TRAP using polyclonal antibodies have been developed (Stepan J.J et al (Biochem, Biophs. Res. Commun. 165:1027-1034, 1989), Kraenzlin M.E et al (Journal of Clinical Endrocrinology and Metabolism 71(2): 442-451, 1990) and Cheung C. K et al (Clin. Chem. 41(5): 679-686, 1995)). Studies using polyclonal antibodies against TRAP have shown that children and postmenopausal women had elevated concentrations of TRAP in their sera (Halleen J. et al. Journal of Bone and Mineral Research 11 (10:1444-1452, 1996).

Chamberlain P. et. Al (Clin. Chem. 41(10): 1495-1499, 1995) describe an assay using monoclonal antibodies against recombinantly produced TRAP.

Based on the finding that TRAP 5b is a more specific biochemical marker for bone resorption than total TRAP, International patent application No. WO 1999/050662, describes an assay for TRAP 5b, using a common phosphatase substrate, paranitrophenylphosphate (pNPP) in a buffer with a defined pH range to maximise the activity of the 5b isoform, while reducing the interference by TRAP 5a.

European Patent No. 1598670 describes acid phosphatase substrates that are suitable for measurement of TRAP 5b, where the relative activity for one of the example substrates, 2-chloro-4-nitrophenylphosphate, for TRAP 5b over TRAP 5a, is only 0.8x greater than that achieved in WO1999/050662 described above.

US Patent No. 7,074,903 and European Patent No. 1359161 (family members) each describe a monoclonal antibody claimed to exhibit specificity for TRAP 5b over TRAP 5a. However the antibody shows equivalent cross-reactivity with recombinant human TRAP5, which is considered to be more similar to the natural TRAP 5a than to TRAP 5b (as isolated from human serum). Thus, the functional specificity of the antibody in the assays described is not significantly better at discriminating between TRAP5a and 5b than the activity assays described in WO1999/050662 or EP1598670.

The known commercial assays are suitable for use in certain clinical applications because the enzymatic activity of TRAP 5a is considerably lower than that of 5b (the clinically useful form). However, by evaluating data published in a range a number of studies, it can be estimated that the interference by 5a in normal samples could be as high as 50%. While this is does not represent a significant difficulty for some clinical applications, where the changes in TRAP 5b levels are substantial, the known methods may lack the ability to distinguish between more subtle changes in TRAP 5b levels.

The substrates pNPP and its 4-halo-substituted derivatives used in the known assays, are not stable in solution. This is not a problem when the assays are performed manually and where the substrate is supplied as a solid to be dissolved immediately before use. However, the instability of the substrates makes it difficult for these compounds to be used on automated immunoassay platforms, which require that a substrate is stable in solution for several weeks.

Patent No. US 3,975,405 describes a monophosphate ester of o-Cresolphthalein and its use as a substrate for measurement of alkaline phosphatase in serum samples with the claimed benefit of avoiding interferences caused by bilirubin and being more sensitive to the intestinal and placental isoforms of alkaline phosphatase than Thymolphthalein monophosphate. The methods described do not distinguish the various isoforms of alkaline phosphatase or acid phosphatase enzymes, or describe its use as a substrate for TRAP5b.

Patent No. US 4,045,290 describes the preparation and use of Thymol Blue monophosphate as a phosphatase substrate for measurement of total alkaline phosphatase in serum and for the measurement of total acid phosphatase in serum under the appropriate pH conditions. The methods described do not distinguish the various isoforms of alkaline phosphatase or acid phosphatase enzymes.

Therefore, there is therefore a requirement for a method for assaying TRAP5b in which the interference by TRAP5a is substantially reduced compared to the existing assays and which can readily be adapted to an automated format.

## BRIEF SUMMARY OF THE DISCLOSURE

The present invention provides the use of a monophosphate ester of a phthalein compound as a substrate for Tartrate-Resistant Acid Phospatase 5b (TRAP 5b).

Herein, a monophosphate ester of a phthalein compound of the present invention is defined as a compound of the following formula:

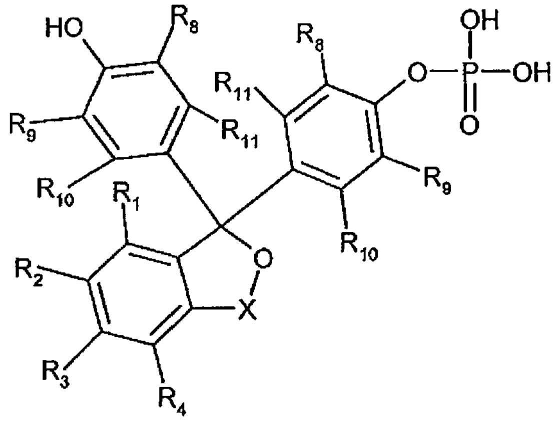

(II)

wherein:

X is CO or $SO_2$;

$R^1$ is selected from hydrogen, halogen, trifluoromethyl, cyano, nitro, $-OR^5$, $-C(O)R^5$,$-C(O)OR^5$ $-OC(O)R^5$, $-S(O)_nR^5$, $-N(R^5)R^6$, $-C(O)N(R^5)R^6$ and $_R{}^7$;

$R^2$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$;

$R^3$ is selected from hydrogen, halogen, trifluoromethyl, cyano, nitro, $-OR^5$, $-C(O)R^5$, - $C(O)OR^5$, $-OC(O)R^5$, $-S(O)_nR^5$, $-N(R^5)R^6$, $-C(O)N(R^5)R^6$ and $R^7$;

$R^4$ is selected from hydrogen, halogen, trifluoromethyl, cyano, nitro, $-OR^5$, $-C(O)R^5$,$-C(O)OR^6$, $-OC(O)R^5$, $-S(O)_nR^5$, $-N(R^5)R^6$, $-C(O)N(R^5)R^6$ and $R^7$;

each $R^8$ is selected from hydrogen, halogen, trifluoromethyl, cyano, nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ and $R^7$;

each $R^9$ is selected from hydrogen, halogen, trifluoromethyl, cyano; nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ and $R^7$;

each $R^{10}$ is selected from hydrogen, halogen, trifluoromethyl, cyano, nitro, $-OR^5$,$-C(O)R^6$, $-C(O)OR^5$, $-OC(O)R^5$, $-S(O)_nR^5$, $-N(R^5)R^6$, $-C(O)N(R^5)R^6$ and $R^7$; and

each $R^{11}$ is selected from hydrogen, halogen, trifluoromethyl, cyano, nitro, $-OR^5$,$-C(O)R^6$, $-C(O)OR^5$, $-OC(O)R^5$, $-S(O)_nR^5$, $-N(R^5)R^6$, $-C(O)N(R^5)R^6$ and $R^7$;
wherein:

n is 0, 1 or 2;

$R^5$ and $R^6$ are each independently hydrogen or $R^7$; and

$R^7$ is selected from hydrocarbyl, $-(CH_2)_k$-heterocyclyl, $-(CH_2)_k$-C(O)-heterocyclyl, any of which is optionally substituted with 1, 2, 3, 4 or 5 substituents independently selected from halogen, cyano, amino, hydroxy, -COOH and esters thereof (e.g. -C(O)-$C_{1-6}$ alkyl), phosphoramidite, $C_{1-6}$ alkyl and $C_{1-6}$ alkoxy; and k is 0, 1, 2, 3, 4, 5 or 6.

Also described but not claimed is a monophosphate ester of a phthalein compound of the present invention is defined as a compound of the following formula

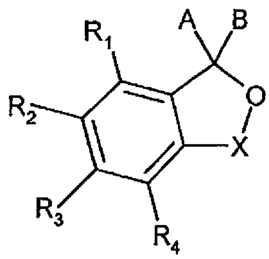

(I)

wherein:

A is substituted or unsubstituted O-phosphorylated para-phenol;

B is substituted or unsubstituted para-phenol phosphate;

X is CO or $SO_2$;

$R^1$ is selected from hydrogen, halogen, trifluoromethyl, cyano, nitro, $-OR^5$, $-C(O)R^6$,$-C(O)OR^5$, $-OC(O)R^5$, $-S(O)_nR^5$, $-N(R^5)R^6$, $-C(O)N(R^5)R^6$ and $R^7$;

$R^2$ is selected from hydrogen, halogen, trifluoromethyl, cyano, nitro, $-OR^5$, $-C(O)R^5$,$-C(O)OR^6$, $-OC(O)R^5$, $-S(O)_nR^5$, $-N(R^5)R^6$, $-C(O)N(R^5)R^6$ and $R^7$;

$R^3$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^6)R^6$, $-C(O)N(R^5)R^6$ and $R^7$; and

$R^4$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$;
wherein:

n is 0, 1 or 2;

$R^5$ and $R^6$ are each independently hydrogen or $R^7$; and

$R^7$ is selected from hydrocarbyl, $-(CH_2)_k$-heterocyclyl, $-(CH_2)_k$-C(O)-heterocyclyl, any of which is optionally substituted with 1, 2, 3, 4 or 5 substituents independently selected from halogen, cyano, amino, hydroxy, -COOH and esters thereof (e.g. -C(O)-$C_{1-6}$ alkyl), phosphoramidite, alkyl and $C_{1-6}$ alkoxy; and k is 0, 1, 2, 3, 4, 5 or 6.

Preferably, a phthalein compound from which a monophosphate ester of the present invention is derived is a phenolphthalein or sulphophthalein, preferably phenolsulphophthalein.

The present invention also provides a method for obtaining a measure of the amount of TRAP 5b in a sample, comprising assaying the activity of TRAP 5b using a monophosphate ester of a phthalein compound as a substrate.

The present invention also provides a method for measuring the bone resorption rate in a subject, comprising obtaining a measure of the amount of TRAP 5b in a sample from the subject by assaying the activity of TRAP 5b using a monophosphate ester of a phthalein compound as a substrate, wherein the amount of TRAP 5b present in the sample is reflective of bone resorption rate.

Also disclosed is a method for diagnosing a disorder associated with change in bone resorption rate in a subject, comprising obtaining a measure of the amount of TRAP 5b present in a sample of the subject by assaying the activity of TRAP 5b using a monophosphate ester of a phthalein compound as a substrate, wherein an abnormal amount of TRAP 5b is indicative of the subject having the disorder.

The present invention also provides a method of monitoring the effect of a drug for the prevention or treatment of a disorder associated with change in bone resorption rate in a subject taking the drug, comprising (a) obtaining a measure of the amount of TRAP 5b present in a sample of the subject by assaying the activity of TRAP 5b using a monophosphate ester of a phthalein compound as a substrate; and (b) comparing the amount of TRAP 5b in the sample to the amount of TRAP 5b in a control sample.

The present invention also provides a method of screening for susceptibility of a subject to a disorder associated with change in bone resorption rate, comprising obtaining a measure of the amount of TRAP 5b present in a sample of

the subject by assaying the activity of TRAP 5b using a monophosphate ester of a phthalein compound as a substrate, wherein an abnormal amount of TRAP 5b is indicative of susceptibility to such a disorder.

Preferably, the methods of the invention comprise (a) binding total TRAP present in a sample to an antibody specific for either total TRAP, TRAP 5a or TRAP 5b; (b) eluting an unbound fraction; and (c) obtaining a measure of the amount of TRAP 5b in the sample either by assaying the activity of TRAP 5b bound to the antibody or the amount of TRAP 5b in the unbound fraction, using a monophosphate ester of a phthalein compound as a substrate.

Preferably, assaying the activity of TRAP 5b in a sample comprises the sequential steps of:

(a) incubating the sample with a monophosphate ester of a phthalein compound as a substrate to allow TRAP 5b to metabolise the substrate;

(b) stopping the metabolism;

(c) optionally adjusting the pH of the sample;

(d) measuring a change in characteristic of the substrate resulting from metabolism by TRAP 5b.

Also provided are kits for carrying out a method of the invention, comprising a monophosphate ester of a phthalein compound, and optionally an antibody which is specific for total TRAP, TRAP 5a or TRAP 5b.

Also provided is the use of a monophosphate ester of a phthalein compound as an indicator of bone resorption rate by acting as a substrate for TRAP 5b. Preferably, there is provided the use of a monophosphate ester of a phthalein compound as an indicator of bone resorption rate by contacting the monophosphate ester with a sample from a subject and subsequently measuring any change in a characteristic of the said monophosphate ester. Preferably there is provided the use of a monophosphate ester of a phthalein compound as an indicator of bone resorption rate by acting as a substrate for TRAP 5b, in a method the invention.

BRIEF DESCRIPTION OF THE DRAWINGS

Figure 1 shows (a) the course of PTMP hydrolysis by TRAP5a; (b) the course of PTMP hydrolysis by TRAP5b; (c) the course of TTMP hydrolysis by TRAP5a; (d) the course of TTMP hydrolysis by TRAP5b. The legend within each chart shows the amount of TRAP5 used per assay.

Figure 2 shows the pH vs activity profiles of TRAP5a and TRAP5b using PTMP substrate.

Figure 3 shows the fractionation of human serum-derived TRAP5a and TRAP5b on CM-Sepharose.

Figure 4 shows the correlation between serum TRAP5 concentrations measured by a pNPP-based assay and a PTMP-based assay.

Figure 5 shows the results of an assay for TRAP 5a and TRAP 5b using p-nitrophenylphosphate (pNPP) and phenolphthalein monophosphate as substrates.

DETAILED DESCRIPTION

Herein, TRAP refers to Tartrate-Resistant Acid Phosphate, which is a member of the type 5 class of acid phosphatases. Unless otherwise stated, "TRAP" or total TRAP includes both isoforms of the enzyme, namely TRAP 5a and TRAP 5b. TRAP 5b is the isoform of the TRAP enzyme which lacks sialic acid in its carbohydrate chain, and exhibits optimum enzyme activity on pNPP substrate between pH 5.7 and 6.1: TRAP 5a is the form of the enzyme which has the sialic acid residue in its carbohydrate chain and exhibits optimum enzyme activity on pNPP at about pH 4.9. The terms TRAP, TRAP5a

Also provided are kits for carrying out a method of the invention, comprising a monophosphate ester of a phthalein compound, and optionally an antibody which is specific for total TRAP, TRAP 5a or TRAP 5b.

Also provided is the use of a monophosphate ester of a phthalein compound as an indicator of bone resorption rate by acting as a substrate for TRAP 5b. Preferably, there is provided the use of a monophosphate ester of a phthalein compound as an indicator of bone resorption rate by contacting the monophosphate ester with a sample from a subject and subsequently measuring any change in a characteristic of the said monophosphate ester. Preferably there is provided

the use of a monophosphate ester of a phthalein compound as an indicator of bone resorption rate by acting as a substrate for TRAP 5b, in a method the invention.

BRIEF DESCRIPTION OF THE DRAWINGS

Figure 1 shows (a) the course of PTMP hydrolysis by TRAP5a; (b) the course of PTMP hydrolysis by TRAP5b; (c) the course of TTMP hydrolysis by TRAP5a; (d) the course of TTMP hydrolysis by TRAP5b. The legend within each chart shows the amount of TRAP5 used per assay.

Figure 2 shows the pH vs activity profiles of TRAP5a and TRAP5b using PTMP substrate.

Figure 3 shows the fractionation of human serum-derived TRAP5a and TRAP5b on CM-Sepharose.

Figure 4 shows the correlation between serum TRAP5 concentrations measured by a pNPP-based assay and a PTMP-based assay.

Figure 5 shows the results of an assay for TRAP 5a and TRAP 5b using p-nitrophenylphosphate (pNPP) and phenolphthalein monophosphate as substrates.

DETAILED DESCRIPTION

Herein, TRAP refers to Tartrate-Resistant Acid Phosphate, which is a member of the type 5 class of acid phosphatases. Unless otherwise stated, "TRAP" or total TRAP includes both isoforms of the enzyme, namely TRAP 5a and TRAP 5b. TRAP 5b is the isoform of the TRAP enzyme which lacks sialic acid in its carbohydrate chain, and exhibits optimum enzyme activity on pNPP substrate between pH 5.7 and 6.1. TRAP 5a is the form of the enzyme which has the sialic acid residue in its carbohydrate chain and exhibits optimum enzyme activity on pNPP at about pH 4.9. The terms TRAP, TRAP5a

Herein, independently is the same or different.

When A is substituted O-phosphorylated para-phenol, suitable substituents include F, Cl, Br, $CH_3$, $CF_3$, $OCF_3$, -C≡N, -OH, $-NO_2$, $-SO_2H$, $-NH_2$, -COOH, $-CONH_2$, $-C_{1-4}$ alkyl (e.g. Me, Et, Pr or Bu) or $-C_{1-4}$ alkoxy (e.g. -OMe, -OEt, -OPr or -OBu) or the like.

When B is substituted para-phenol phosphate, suitable substituents include F, Cl, Br, $CH_3$, $CF_3$, $OCF_3$, -C=N, -OH, $-NO_2$, $-SO_2H$, $-NH_2$, -COOH, $-CONH_2$, $-C_{1-4}$ alkyl (e.g. Me, Et, Pr or Bu) or $-C_{1-4}$ alkoxy (e.g. -OMe, -OEt, -OPr or -OBu) or the like.

In one class of embodiments, the compounds comprise at least one substituent selected from $R^1$ to $R^{11}$ which comprises a reactive functional group which is directly or indirectly linked to another molecule, e.g. a biomolecule, a protein, a polypeptide.

In an embodiment, $R^1$ to $R^4$ are each H, X is CO and $R^8$, $R^9$, $R^{10}$ and $R^{11}$ are as defined in table 1. In another embodiment, $R^1$ to $R^4$ are each H, X is $SO_2$ and $R^8$, $R^9$, $R^{10}$ and $R^{11}$ are as defined in table 2.

In one embodiment, each $R^8$ group is independently bromo, chloro or fluoro and each $R^9$ group is independently methyl, ethyl, iso-propyl or t-butyl. Optionally each $R^8$ group is independently bromo or chloro and each $R^9$ group is independently methyl, ethyl or isopropyl.

In one embodiment, only one, two or three of $R_1$ to $R_{11}$ is other than H.

In one embodiment, each $R^8$ group is independently bromo, chloro or fluoro and each $R^9$ group is independently bromo, chloro or fluoro. Optionally, each $R^8$ group is independently bromo or chloro and each $R^9$ group is independently bromo or chloro.

In one embodiment, each $R^8$ group is independently methyl, ethyl, iso-propyl or t-butyl and each $R^{10}$ group is methyl, ethyl, iso-propyl or t-butyl. Optionally, each $R^8$ group is independently methyl, ethyl or iso-propyl and each $R^{10}$ group is methyl, ethyl or isopropyl.

Preferred monophosphate esters of a phthalein compound in the present invention include phenolphthalein monophosphate, Cresolphthalein monophosphate, Thymolphthalein monophosphates, o-Chlorophenolphthalein monophosphate, Phenol Red monophosphate, o-Cresol Red monophosphate, m-Cresol Purple monophosphate, Bromocresol Purple monophosphate, Bromocresol Green monophosphate, Bromophenol Blue monophosphate, Thymol Blue monophosphate, Bromothymol Blue monophosphate , Xylenol Blue monophosphate, and Chlorophenol Red monophosphate.

Also included as substrates in the present invention are functional derivatives or salts of the monophosphate esters of the phthalein compounds of the present invention, which exhibit a change in characteristic upon metabolism

by TRAP 5b

The monophosphate esters of the phthalein compounds may be produced by esterification of either hydroxyl group of Forumla I. The monophosphate esters of a phthalein compound can be prepared by methods known in the art and described, for example, in US 3,975,405 and US 4,045,290.

A subject may be any vertebrate, preferably a mammal, and most preferably a human being. In embodiments, the subject is female.

A sample from a subject may be any body fluid sample derived from the subject, preferably a blood sample, such as blood plasma or serum. Other suitable body fluid samples include synovial fluid, saliva and urine.

Antibodies against TRAP used in the present invention include antibodies which bind TRAP, total and are not specific for either isoform, as well as antibodies which show preferential binding for a TRAP isoform, and thus are referred to as being specific for TRAP 5a or TRAP 5b. Methods for determining specificity of anti-TRAP antibodies are described in the art, for example in Halleen et al JBMR vol 15 1335 - 1434 (2000). The antibodies used in the present invention may be polyclonal or monoclonal, but are preferably the latter. The antibodies may be raised against TRAP 5b from any species, but preferably from mouse, rat or human. Preferably, the antibody is derived from any suitable animal source, for example mouse, rabbit, sheep, goat using native TRAP 5b as an immunogen, using methods available in the art (Tissjen, (ed) Practice and Theory of Enzyme Immunoassay. Laboratory Techniques in Biochemistry and Molecular Biology, vol 15 chapter 5). Preferred monoclonal antibodies for use in the present invention include 4E6, 01A, and J1 B (Halleen et al JMBR 14 (464-9 (1999)), 14G6 and 9C5 (Janckilla et al Hybridoma 16, 175-82. (1997)).

The invention is based upon using monophosphate esters of phthalein compound as a substrate for TRAP 5b, in order to obtain a measure of the amount of TRAP in a sample, as an indication of bone resorption rate in a subject. This is based upon the observations that monophosphate esters of phthalein compounds are preferentially metabolised by TRAP 5b rather than TRAP 5a, and as such can be used to obtain a specific measure of TRAP 5b activity in a sample. The activity of TRAP 5b on the substrate, also referred to as metabolism of the substrate by TRAP 5b, changes a characteristic of the substrate, and this change is used as a measure of the activity of TRAP 5b and therefore a measure of the amount of TRAP 5b in the sample. The characteristic may be any measurable characteristic, for example a change in structure or function. Preferably, the change is a structural one, and preferably is the removal of a phosphate group from the substrate by TRAP 5b, preferably the removal of phosphate ester group of formula (II). This structural change results in a colour change in the substrate, which can be measured to indicate the amount of TRAP 5b present.

Thus, obtaining a measure of the amount of TRAP 5b comprises assaying the activity of TRAP 5b on a substrate. The assay comprises incubating a sample from a subject with a substrate of the invention.

Preferably, the assay comprises first binding TRAP in the sample to an antibody, to isolate TRAP from the sample. Depending upon the specificity of the antibody used, either total TRAP, TRAP 5a or TRAP 5b may be isolated. TRAP 5b activity can then be measured either by adding the substrate to an unbound fraction or a bound fraction of the sample, depending upon the specificity of antibody used. Where a non-isoform specific antibody is used, other methods may be additionally employed for determining TRAP 5b activity specifically, such as performing the activity assay under conditions which favour TRAP 5b activity. Preferably, therefore, the step of assaying the activity of TRAP 5b comprises either i) binding to a TRAP 5a specific antibody and assaying the activity of the non-bound fraction comprising TRAP 5b on the substrate; (ii) binding to a TRAP 5b specific antibody and assaying the activity of the bound fraction comprising TRAP 5b on the substrate; or (iii) binding to a non-isoform specific TRAP antibody; assaying the activity of the bound fraction comprising TRAP 5a and TRAP 5b on the substrate under conditions which favour TRAP 5b activity. In a preferred embodiment to further increase the specificity of the assay for TRAP 5b activity, (i) and/or (ii) above may also be carried out under conditions which favour TRAP 5b activity.

Conditions which influence TRAP 5b activity over TRAP 5a activity include pH. For example, the assay may be performed at a pH preferential for TRAP 5b activity rather than TRAP 5a activity. Thus, preferably, the step of assaying the activity of TRAP 5b is carried out at pH 6.8 to 7.6, preferably pH 6.8 to 7.4, and most preferably pH 7-7.2.

Preferably, the metabolism of the substrate is stopped by altering the reaction conditions, such as by adding a reagent, such as sodium hydroxide, hydrochloric acid, disodium hydrogen orthophosphate, sodium dihydrogen orthophosphate or 1 M glycine/NaOH pH 11.

The change in characteristic of the substrate due to metabolism of TRAP 5b may be measured using any suitable means. Where the change results in a colour change, this is preferably measured using a spectrophotometer. The value obtained can be used as an indication of the amount of activity by TRAP 5b and therefore can be used as a measure of the amount of TRAP 5b in the sample. This value may be translated into amount of TRAP 5b by comparison to a calibrated standard or chart.

Where a spectrophotometer is used to measure change in colour of the substrate, the absorbance is measured at a wavelength at which the phthalein product has a maximum absorbance. Preferably, the absorbance is measured at 450nm, 540nm, 550nm, 592nm or 595nm.

Some substrates may require adjustment of the pH in order for any colour change in the sample to be observed. Thus, the methods of the invention may include adjusting the pH of the sample prior to measuring any colour change

in the sample. The pH may be adjusted by adding an appropriate amount of acid or alkali to bring the pH of the sample to that at which any colour change for the particular substrate is observed. The pH's at which the colour change of substrate is observed are provided in "Data for Biochemical Research 3rd Edition, RMC Dawson et al, Clarendon Press, Oxford 1989.

Thus, the methods of the present invention may further provide for obtaining an absolute value of the amount of TRAP 5b present, by comparing the measure of TRAP 5b obtained to a calibrated standard or chart. An absolute value is the amount of TRAP 5b measured in units of micromolecules of substrate hydrolysed per minute.

Preferably, multiple samples may be assayed simultaneously on, for example, an array, chip, or multi-well plate. The assays of the present invention are preferably carried out on an immobilising matrix on an automated platform. Preferably, the immobilising matrix is antibody coated. Most preferably, where a matrix is used, it comprises magnetic beads to which the antibody is coated, to enable a high throughput analysis of samples for example by a robotic or otherwise automated instrument.

A disorder related to changes in bone resorption rate includes any disorder where the rate of bone resorption is abnormal, typically resulting in abnormal bone density. Such disorders may include osteoporosis, Pagets disease of bone, multiple myeloma; metabolic bone diseases, metastases, bone lesions, osteoarthritis, rheumatoid arthritis, and cardiovascular disorders, particularly those associated with artherosclerotic plaque formation. A change in bone resorption rate means a change in the degree to which bone is broken down or absorbed, which may therefore affect the density of the bone.

The bone resorption rate can be determined by comparison of either the amount of TRAP 5b in a sample or the activity of TRAP 5b with a calibrated standard or chart for bone resorption rates. The bone resorption rate obtained can be used to diagnose a disorder associated with a change in bone resorption rate or determine susceptibility to such a disorder. In a preferred embodiment, the methods of the present invention comprise comparing the measure of TRAP in a sample with a chart to directly correlate said measure with susceptibility to, or presence of, a specific disorder.

By 'diagnosing a disorder' is meant determining the presence of a disorder by assessing the bone resorption rate in a subject. The method may also include identifying the presence or absence of other symptoms known to be associated with a particular disorder, or bone resorption rate.

By determining susceptibility to a disorder' is meant determining the likely onset of the disorder in a subject by assessing the bone resorption rate. The method may additionally compare identifying any other indicators of likely onset of said disorder.

The methods of the invention which relate to monitoring the effectiveness of a drug for use in the treatment or prevention of a disorder associated with abnormal bone resorption rate comprise comparing the amount of TRAP 5b activity in a sample from a subject to whom the drug has been administered with a control sample from a subject who has not received the drug. Thus, a control sample may be defined as being taken from a subject who has not received the drug to be tested, and preferably whose TRAP 5b activity is abnormal, and more preferably has the same disorder as the subject on whom the drug is being tested. The subject may be the same or different as the subject to whom the drug has been administered, but is preferably the same. Where the subject is the same, the control sample is preferably taken from the subject before any administration of the drug. In the method of monitoring the effectiveness of a drug, a control sample may be compared to one or more samples taken from a subject after administration of the drug, preferably at suitable time intervals. The effectiveness of the drug is determined by assessing any change in bone resorption rate upon administration of the drug in a sample from the subject will determine its effectiveness.

A kit according to the present invention comprises a monophosphate ester of a phthalein compound, and optionally an antibody which binds TRAP, TRAP 5a and/or TRAP 5b. Preferably, these are provided as separate reagents for use in an assay of the invention. The kits may also provide reagents for use in the methods of the invention, including those described herein, instructions for use, calibration charts, a substrate on which the assay is performed, pipettes, dispensers, and/or cuvettes.

The embodiments described herein in relation to the first aspect of the invention apply to the other aspects, *mutatis mutandis.*

Throughout the description and claims of this specification, the words "comprise" and "contain" and variations of the words, for example "comprising" and "comprises", means "including but not limited to", and is not intended to (and does not) exclude other moieties, additives, components, integers or steps.

Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith.

EXAMPLES

Substituted phenolphthalein monophosphate esters or salts of the form -

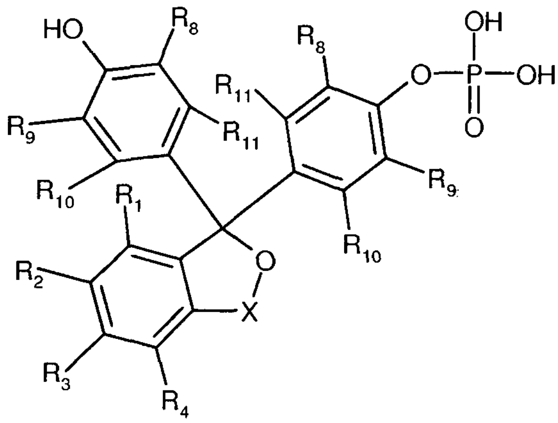

wherein X is CO or $SO_2$;
$R^8$ selected from H, alkyl (Methyl, Ethyl, isopropyl), halogen (Bromo, Chloro, Fluoro), or Alkoxy (Methoxy, ethoxy, i-propoxy, t-butoxy);
$R^9$ selected from H, alkyl (Methyl, Ethyl, isopropyl), halogen (Bromo, Chloro, Fluoro), or Alkoxy (Methoxy, ethoxy, i-propoxy, t-butoxy);
$R^{10}$ selected from H or Methyl;
$R^{11}$ selected from H or Methyl;
$R^3$ selected from H, halogen (Bromo, Chloro, Fluoro) or Alkoxy (methoxy, ethoxy, i-propoxy, t-butoxy);
$R^1$, $R^2$ and $R^4$ are H.

Examples include substituted "phenolphthalein-like" monophosphate esters or salts, wherein X is C=O, $R^1$, $R^2$, $R^4$ are H, and $R^3$, $R^8$-$R^{11}$ are defined in Table 3;

Table 3.

| $R^8$ | $R^9$ | $R^{10}$ | $R^{11}$ | $R^3$ | |
|---|---|---|---|---|---|
| H | H | H | H | H | Phenolphthalein monophosphate |
| Me | H | H | H | H | o-Cresolphthalein monophosphate |
| iso-propyl | H | Me | H | H | Thymolphthalein monophosphate |
| Cl | H | H | H | H | o-Chlorophenolphalein monophosphate |

Examples include substituted "phenolsulfonphthalein-like" monophosphate esters or salts, wherein X is $SO_2$, $R^1$, $R^2$, $R^4$ are H, and $R^3$, $R^8$-$R^{11}$ are defined in table 4;

Table 4.

| $R^8$ | $R^9$ | $R^{10}$ | $R^{11}$ | $R^3$ | |
|---|---|---|---|---|---|
| H | H | H | H | H | Phenol Red monophosphate |
| Me | H | H | H | H | o-Cresol Red monophosphate |
| H | H | H | Me | H | m-Cresol Purple monophosphate |
| Br | Me | H | H | H | Bromocresol Purple monophosphate |
| Br | Br | Me | H | H | Bromocresol Green monophosphate |
| Br | Br | H | H | H | Bromophenol Blue monophosphate |

(continued)

| $R^8$ | $R^9$ | $R^{10}$ | $R^{11}$ | $R^3$ | |
|---|---|---|---|---|---|
| iso-propyl | H | Me | H | H | Thymol Blue monophosphate |
| iso-propyl | Br | Me | H | H | Bromothymol Blue monophosphate |
| Me | H | Me | H | H | Xylenol Blue monophosphate |
| Cl | H | H | H | H | Chlorophenol Red monophosphate |

The Monophosphate esters for use in the present invention can be prepared by methods such as described in US 3,975,405 and US 4,045,290. Phenolphthalein monophosphate was purchased from Sigma-Aldrich (part P5758) and thymolphthalein monophosphate was purchased from TCI (part T0863).

**Example 1 Demonstration that Phenolphthalein monophosphate and Thymolphthalein monophosphate are substrates for TRAP5a and TRAP5b.**

Microtitre plates (Nunc Maxisorp) were coated with mouse anti-TRAP5 (clone O1A), from a solution of a 3.0mg/L purified Mab IgG in 10mM phosphate buffered saline (PBS), 100μL per well and incubated overnight at room temperature. The coated plates were washed three times with 10mM phosphate buffered saline, 0.05% Tween 20. A stabilising reagent of 5% lactose, 0.5% BSA was added, 250μL per well, incubated for at least 10 minutes, then the liquid aspirated. The coated plates were dried overnight at 35°C then stored at 2-8°C protected from moisture.

Serum-derived TRAP5a and TRAP5b were separated by ion exchange chromatography using a modification of the procedure described by Lam et al, Clin. Chem. 24(7):1105-1108, 1978. 100-500 mL human serum was adjusted to around pH 5.0 with 100% acetic acid, and optionally diluted with an equal volume of 10 mM sodium phosphate buffer, pH 4.9. After clarification by centrifugation and filtering, the acidified serum was applied to a 1 cm x 100-150 cm column of CM-Sepharose (Sigma, part No CCF 100) and the column was developed with a linear NaCl gradient, from 150-800 mM. Fractions containing TRAP5a and TRAP5b activity were identified using a non-capture assay essentially as described by Lam et al Clin. Chem. 24(7):1105-1108, 1978. In some instances the fractions were further assayed for TRAP5 by the immunoassays described below. Fractions containing peak levels of TRAP5a or TRAP5b were separately pooled for further analysis.

The TRAP5 protein concentration in the TRAP5a and TRAP5b preparations was determined by a sandwich immunoassay. 100-200μL sample per well was applied to O1A-coated microtitre plates and incubated overnight at 4°C. The plate was washed three times with 10mM phosphate buffered saline, 0.05% Tween 20, and 100-200μL of a solution containing the rabbit anti-TRAP5 antibody at a dilution of 1:4000 was added per well. After 60 min incubation at ambient temperature the plate was washed as above and 100-200μL of a solution containing anti-rabbit IgG-Horseradish peroxidase conjugate at a dilution of 1:5000 was added and incubated for 30 min at ambient temperature. After further washing as described above 200μL of TMB (BioFx Inc, part TMBW-0100-01) was added and incubated for 30 min at ambient temperature. The reactions were stopped by the addition of 100μL 0.5M HCl and the absorbance at 450 nm was measured. TRAP5 protein concentrations were calculated by reference to a calibration curve prepared from baculovirus-derived recombinant human TRAP5.

The ability of TRAP5a and TRAP5b to use Phenolphthalein monophosphate (PTMP) and Thymolphthalein monophosphate (TTMP) as substrates was demonstrated by applying a 100μL sample per well to O1A-coated microtitre plates and incubating at ambient temperature for 60 min before washing three times with 10mM phosphate buffered saline, 0.05% Tween 20. To test for activity towards PTMP, 100μL of a solution containing 6 mg/mL PTMP dissolved in 125 mM MOPS buffer, pH 7.0 was added per well and incubated for 0-40 min at 37°C. The reactions were stopped at the times indicated by the addition of 50μL per well of 1M glycine/NaOH, pH11 and the absorbance at 550 mm was measured. To test for activity towards TTMP, 100μL of a solution containing 5 mg/mL TTMP dissolved in 80 mM MOPS buffer, 10% ethanol, pH 7.0 was added per well and incubated for 0-40 min at 37°C The reactions were stopped at the times indicated by the addition of 50μL per well of 0.25 M NaOH and the absorbance at 592 mm was measured. The results are shown in Figure 1.

Since the enzyme activity measurements were made at pH 7.0, it was surprising to find that both TRAP5a and TRAP5b, which are isoforms of an acid phosphatase, could readily hydrolyze the PTMP and TTMP substrates at neutral pH, as judged by the time-dependent increase in absorbance at 550 nm and 592 nm respectively. In all cases the reaction rate increased with the amount of TRAP5a or TRAP5b used in the assay. Furthermore, it is evident by comparing the data in Fig 1 a with that in 1 b, that by using PTMP as a substrate at pH 7.0, the specific activity of TRAP5b is approximately 50 times that of TRAP5a. Similarly, it is evident by comparing the data in Fig 1c with that in 1 d, that using by TTMP as a substrate at pH 7.0, the specific activity of TRAP5b is approximately 70 times that of TRAP5a.

**Example 2. pH profiles of TRAP5a and TRAP5b using PTMP as substrate.**

Since TRAP5 is an acid phosphatase that normally is only active under acidic conditions, the relationship between TRAP5a and TRAP5b enzyme activity and substrate pH using PTMP as substrate over a range of pH values was investigated. The TRAP5 assays were performed essentially as described for Example 1, except that the PTMP substrate was dissolved at 6 mg/mL in a series of 100 mM MES or 100 mM MOPS buffers to give the pH values indicated. The results are shown in Figure 2.

It was surprising to find that the TRAP5b exhibits maximal activity with PTMP as substrate at a pH of around pH7.0, and that the corresponding value for TRAP5a is around pH 6.0. These data are in marked contrast to the results of all previous workers in this area who have reported consistently reported acidic pH optima for both forms of TRAP5. For example, the data disclosed in WO1999/050662 show that the pH optima for TRAP5a and TRAP5b using pNPP are around 4.9 and 5.9 respectively. Similarly the data disclosed in EP1598670 show that the pH optimum for TRAP5b using 2-chloro-4-nitrophenylphosphate is 6.4.

**Example 3. Comparison of a Phthalein Monophosphate-based assay for TRAP5b with an existing commercial TRAPb assay.**

TRAP5a and TRAP5b preparations were prepared and quantified with respect to TRAP5 mass as described in Example 3. A set of TRAP5 calibrators was prepared by incubating 225 μg baculovirus-derived recombinant human TRAP with 12 U chymotrypsin for 6 h at ambient temperature, preparing a series of dilutions from this material and assigning TRAP5 concentrations (in U/L) to these dilutions using the commercial TRAP5b immunoassay based on WO1999/050662. The TRAP5a and TRAP5b preparations were assayed by a commercial TRAP5b immunoassay based on WO1999/050662 which uses pNPP as substrate at pH 6.1 with the modification that the chymotrypsin-treated calibrators described above were substituted for those provided by the manufacturer. The TRAP5a and TRAP5b preparations were also assayed using the PTMP- and TTMP-based immunoassays described in Example 3, with the additional modification that the TRAP5 concentration (in U/L) was calculated by reference to a calibration curve prepared from the chymotrypsin-treated calibrators described above. The specific activities, in U/μg, of the TRAP5a and TRAP5b preparations with each substrate were calculated and are presented in Table 1.

**Table 1: Specific activity of TRAP5a and TRAP5b using pNPP, PTMP or TTMP as substrate**

| | TRAP5 Specific Activity U/μg | | |
|---|---|---|---|
| | **5A** | **5B** | **Ratio 5B:5A** |
| **pNPP** | 0.177 | 1.60 | 9.04 |
| **PTMP** | 0.035 | 1.68 | 47.5 |
| **TTMP** | 0.035 | 2.3 | 65.3 |

The results for the pNPP substrate are in agreement with the data disclosed in WO1999/050662, the specific activity of TRAP5b is 9-fold greater that that of TRAP5a and the results also reveal that the corresponding values for the PTMP and TTMP-based assays are 47.5-fold and 65.3-fold respectively. Therefore, the specificities of the PTMP and TTMP-based assays for TRAP5b over TRAP5a are some 4.75-fold and 6.5-fold higher than the commercial pNPP based assay. The results in Table 1- also show that this improvement in specificity is due to a decrease in the amount of TRAP5a detected by the PTMP and TTMP-based assays rather than their detecting an increased amount of TRAP5b.

**Example 4. Demonstration of specificity of a PTMP-based assay.**

TRAP5a and TRAP5b from normal human serum were separated on a CM-Sepharose column and the fractions assessed for TRAP5b using the pNPP and PTMP-based assays described in Example 3. The results are shown in Figure 3.

The results show that the pNPP-based assay detects two distinct populations of TRAP5. The first, with a peak at fraction 53 is TRAP5a, while the second, with a peak at fraction 61 is TRAP5b. This data set clearly illustrates the major disadvantage of the existing commercial TRAP5b immunoassays, that they detect substantial amounts of TRAP5a, in this example around 40% of the material measured as TRAP5b is actually TRAP5a. It can however, be clearly seen that the PTMP-based assay detects substantially less TRAP5a than the pNPP based assay, Indeed, as judged by the quantities measured in the peak TRAP5a fraction 53, in this example the PTMP-based assay detects only 16% of the TRAP5a detected by the pNPP-based assay. It is also apparent that the PTMP- and pNPP based assays detect similar

amounts of TRAP5b.

**Example 5. Correlation of serum TRAP5b levels measured with pNPP-based and PTMP-based assays.**

The amounts of serum TRAP5b measured by the commercial pNPP-based assay and by the PTMP-based assay were compared using a panel of 32 serum samples. The samples were assayed using the commercial pNPP-based assay exactly as supplied by the manufacturer, and using the PTMP-based assay described in Example 4. The correlation between the two assays is shown in Figure 4.

The results show that there is an excellent correlation between the PTMP- and the pNPP based assays ($r^2$=0.96). The relationship between the two datasets is:

**(PTMP-based assay) = 1.09x(pNPP-based assay) – 0.62.**

This show that the values obtained with the PTMP-based assay are slightly lower than those obtained using the pNPP-based assay, by around 0.62 U/L. This difference can be explained by the PTMP-based assay detecting less TRAP5a than the pNPP-based assay. The data therefore demonstrate the utility of the PTMP-based assay in determining TRAP5b concentrations in clinically relevant samples.

**Example 6**

Tartrate-resistant acid phosphatase 5b (TRAP5b) is secreted exclusively by osteoclasts. The level of TRAP5b in serum is a measure of osteoclast abundance and hence an indirect indicator of bone resorption. Measurement of TRAP5b in serum is complicated by the presence of an approximately 10-fold excess (by mass) of TRAP5a, produced principally by macrophages and dendritic cells. Existing commercial immunoassays for TRAP5b involve the capture of TRAP5 by an immobilised antibody; followed- by an activity measurement at a pH where the specific activity of TRAP5b is 10-17x that of the TRAP5a. In seeking to transfer the BoneTRAP™ assay onto the 3x3 automated platform, we encountered problems relating to the stability of the substrate pNPP in liquid phase.

This led us to examine the utility of phthalein monophosphates as TRAP5b substrates. We examined the abilities of serum-derived TRAP5a and TRAP5b (separated by chromatography on CM-Sepharose), and baculovirus-derived recombinant TRAP5, to hydrolyse phthalein monophosphates. All TRAP5 preparations hydrolised phenolphthalein monophosphate (PTMP) and thymolphthalein monophosphate (TTMP). We observed dramatic and surprising differences between the pH optima of these preparations with the phthalein monophosphate substrates, compared to those determined with pNPP. For example, using PTMP, the pH optima of TRAP5a and TRAP5b were 6.0 and 7.0 respectively. These values are markedly higher than those obtained using pNPP, (4.9 and 5.9 respectively).

We next established an immunoassay for TRAP5b in which the enzyme activity was measured using PTMP at pH 7.0 and compared this with an immunoassay using pNPP at pH 6.1. Typical specific activities obtained for the native TRAP5 forms were: TRAP5a (PTMP); 0.035 U/μg: TRAP5a (pNPP); 0.177 U/μg: TRAP5b (PTMP); 1.68 U/μg: TRAP5b (pNPP); 1.60 U/μg. These data indicate that while the PTMP and pNPP-based assays detect similar amounts of TRAP5b, the PTMP-based assay detects only 20% of the TRAP5a detected by the pNPP-based assay. On a panel of 34 patient samples there was excellent correlation between the PTMP and pNPP-based assays, $R^2$ = 0.96. Moreover, the values obtained with the PTMP based assay were slightly lower than those obtained using the pNPP-based assay (y=1.09x-0.62), consistent with this assay detecting less TRAP5a.

Our results demonstrate that phthalein monophosphates are more specific substrates for TRAP5b than pNPP and related compounds by providing an assay where the specific activity of TRAP5b is 47.5x that of TRAP5a. They also reveal that TRAP5 is not intrinsically an acid phosphatase, and that the pH optimum of the enzyme is determined at least in part by the nature of the substrate.

**Example 7**

Where phthalein monophosphate esters were not available commercially, the monophosphate esters were prepared by the method of Bulbenko & Woodbridge (US Patent No 4,045,290) by reaction of the appropriate phthalein compound (from Sigma-Aldrich) with phophorus oxychloride (CAS 10025-87-3), followed by hydrolysis and purification. The monophosphate esters were further purified before use by HPLC chromatography to remove un-reacted phthalein dye. HPLC column 10mmx150mm ACE 5 C18 (Advance Chromatography Technologies, UK), load solvent composition, 30% methanol and 70% water containing 0.1% trifluoroacetic acid (TFA); product was eluted by a gradient from 30% methanol 0.1%TFA to 100% methanol 0.1%TFA at 3mL/minute over 45 minutes..

**Example 8**

Phthalein monophosphate esters prepared in Example 7 were diluted to 0.47mg/mL in 100mM MES pH7.4 buffer and applied to an assay as in Example 1.

| Monophosphate ester (0.47mg/mL) | Detection Wavelength | 5a Activity | 5b Activity | Ratio of 5b:5a activity |
|---|---|---|---|---|
| Bromocresol Green | 590nm | 0.0036 | 0.2636 | 73.2 |
| Bromocresol Purple | 590nm | 0.0074 | 0.4755 | 64.3 |
| Chlorophenol Red | 572nm | 0.0067 | 0.3558 | 53.1 |
| Phenol Red | 550nm | 0.0067 | 0.3558 | 53.1 |
| Phenolphthalein | 550nm | 0.0110 | 0.3664 | 33.3 |

## Claims

1. The use of a monophosphate ester of a phthalein compound as a substrate to obtain a specific measure of activity and/or amount of Tartrate-Resistant Acid Phospatase 5b (TRAP 5b) in a sample, wherein the phthalein compound is a compound having the formula:

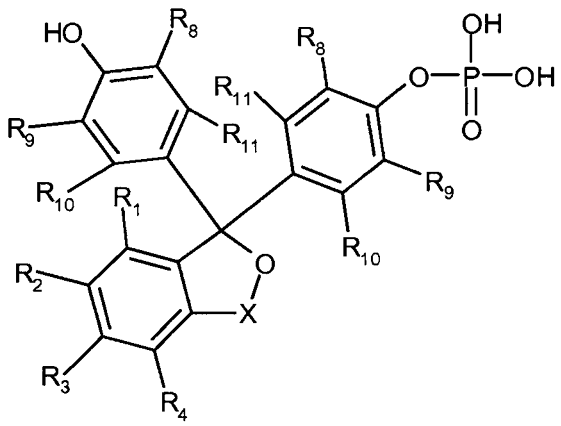

(II)

wherein:

X is CO or $SO_2$;
$R^1$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$;
$R^2$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$;
$R^3$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$;
$R^4$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$;
each $R^8$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$;
each $R^9$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$;
each $R^{10}$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$; and
each $R^{11}$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$;

wherein:

n is 0, 1 or 2;
$R^5$ and $R^6$ are each independently hydrogen or $R^7$; and
$R^7$ is selected from hydrocarbyl, $-(CH_2)_k$-heterocyclyl, $-(CH_2)_k$-C(O)-heterocyclyl, any of which is optionally substituted with 1, 2, 3, 4 or 5 substituents independently selected from halogen, cyano, amino, hydroxy, -COOH and esters thereof (e.g. -C(O)-$C_1$-$_6$ alkyl), phosphoramidite, $C_{1-6}$ alkyl and $C_{1-6}$ alkoxy; and k is 0, 1, 2, 3, 4, 5 or 6.

**2.** A method for obtaining a measure of the amount of TRAP 5b in a sample, comprising assaying the activity of TRAP 5b using a monophosphate ester of a phthalein compound as a substrate, wherein the phthalein compound is a compound having the formula:

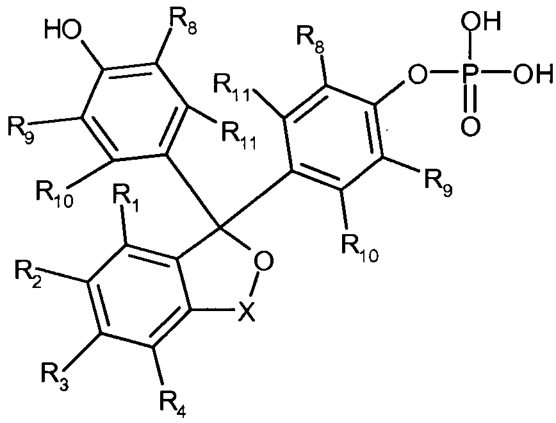

(II)

wherein:

X is CO or $SO_2$;
$R^1$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$;
$R^2$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$;
$R^3$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$;
$R^4$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$;
each $R^8$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$;
each $R^9$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$;
each $R^{10}$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$; and
each $R^{11}$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$;

wherein:

n is 0, 1 or 2;
$R^5$ and $R^6$ are each independently hydrogen or $R^7$; and
$R^7$ is selected from hydrocarbyl, $-(CH_2)_k$-heterocyclyl, $-(CH_2)_k$-C(O)-heterocyclyl, any of which is optionally substituted with 1, 2, 3, 4 or 5 substituents independently selected from halogen, cyano, amino, hydroxy, -COOH and esters thereof (e.g. -C(O)-$C_{1-6}$ alkyl), phosphoramidite, $C_{1-6}$ alkyl and $C_{1-6}$ alkoxy; and k is 0, 1, 2, 3, 4, 5 or 6.

**3.** A method for measuring the bone resorption rate in a subject, comprising obtaining a measure of the amount of TRAP 5b in a sample from the subject by assaying the activity of TRAP 5b using a monophosphate ester of a

phthalein compound as a substrate, wherein the amount of TRAP 5b present in the sample is reflective of bone resorption rate, wherein the phthalein compound is a compound having the formula:

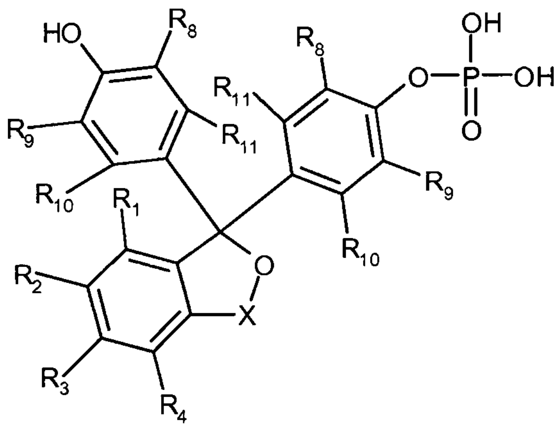

(II)

wherein:

X is CO or $SO_2$;
$R^1$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$;
$R^2$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$;
$R^3$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$;
$R^4$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$;
each $R^8$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$;
each $R^9$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$;
each $R^{10}$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$; and
each $R^{11}$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$;

wherein:

n is 0, 1 or 2;
$R^5$ and $R^6$ are each independently hydrogen or $R^7$; and
$R^7$ is selected from hydrocarbyl, $-(CH_2)_k$-heterocyclyl, $-(CH_2)_k$-C(O)-heterocyclyl, any of which is optionally substituted with 1, 2, 3, 4 or 5 substituents independently selected from halogen, cyano, amino, hydroxy, -COOH and esters thereof (e.g. -C(O)-$C_{1-6}$ alkyl), phosphoramidite, $C_{1-6}$ alkyl and $C_{1-6}$ alkoxy; and k is 0, 1, 2, 3, 4, 5 or 6.

**4.** A method for diagnosing susceptibility to disorders associated with change in bone resorption rate in a subject, comprising obtaining a measure of the amount of TRAP 5b present in a sample of the subject by assaying the activity of TRAP 5b using a monophosphate ester of a phthalein compound as a substrate, wherein an abnormal amount of TRAP 5b is indicative of the subject having the disorder, wherein the phthalein compound is a compound having the formula:

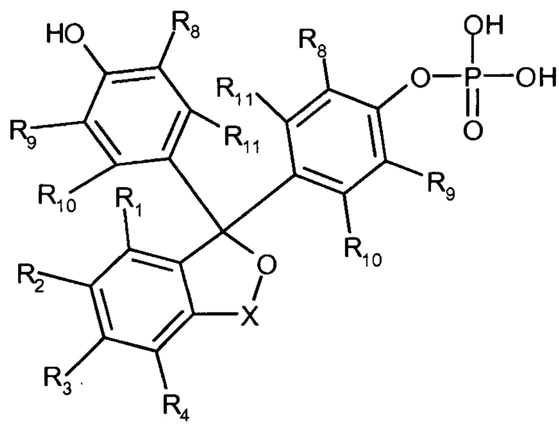

(II)

wherein:

X is CO or $SO_2$;
$R^1$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$;
$R^2$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$;
$R^3$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$;
$R^4$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$;
each $R^8$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$;
each $R^9$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$;
each $R^{10}$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$; and
each $R^{11}$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$;

wherein:

n is 0, 1 or 2;
$R^5$ and $R^6$ are each independently hydrogen or $R^7$; and
$R^7$ is selected from hydrocarbyl, $-(CH_2)_k$-heterocyclyl, $-(CH_2)_k$-C(O)-heterocyclyl, any of which is optionally substituted with 1, 2, 3, 4 or 5 substituents independently selected from halogen, cyano, amino, hydroxy, -COOH and esters thereof (e.g. -C(O)-$C_{1-6}$ alkyl), phosphoramidite, $C_{1-6}$ alkyl and alkoxy; and k is 0, 1, 2, 3, 4, 5 or 6.

**5.** A method of monitoring the effect of a drug for the prevention or treatment of a disorder associated with change in bone resorption rate in a subject taking the drug, comprising (a) obtaining a measure of the amount of TRAP 5b present in a sample of the subject by assaying the activity of TRAP 5b using a monophosphate ester of a phthalein compound as a substrate; and (b) comparing the amount of TRAP 5b in the sample to the amount of TRAP 5b in a control sample, wherein the phthalein compound is a compound having the formula:

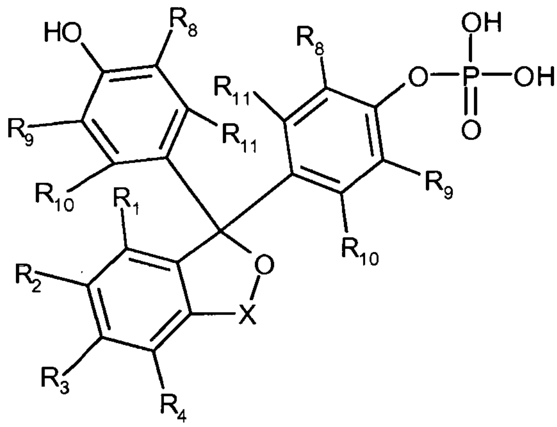

(II)

wherein:

X is CO or $SO_2$;
$R^1$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$;
$R^2$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$;
$R^3$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$;
$R^4$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$;
each $R^8$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$;
each $R^9$ is selected from hydrogen, halogen, trifluoromethyl, cyano, nitro, $-OR^5$, $-C(O)R^5$,$-C(O)OR^5$, $-OC(O)R^5$, $-S(O)_nR^5$ $-N(R^5)R^6$, $-C(O)N(R^5)R^6$ and $R^7$;
each $R^{10}$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$; and
each $R^{11}$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$;

wherein:

n is 0, 1 or 2;
$R^5$ and $R^6$ are each independently hydrogen or $R^7$; and
$R^7$ is selected from hydrocarbyl, $-(CH_2)_k$-heterocyclyl, $-(CH_2)_k$-C(O)-heterocyclyl, any of which is optionally substituted with 1, 2, 3, 4 or 5 substituents independently selected from halogen, cyano, amino, hydroxy, -COOH and esters thereof (e.g. $-C(O)-C_{1-6}$ alkyl), phosphoramidite, $C_{1-6}$ alkyl and $C_{1-6}$ alkoxy; and k is 0, 1, 2, 3, 4, 5 or 6.

**6.** A method of screening for susceptibility of a subject to a disorder associated with change in bone resorption rate, comprising obtaining a measure of the amount of TRAP 5b present in a sample of the subject by assaying the activity of TRAP 5b using a monophosphate ester of a phthalein compound as a substrate, wherein an abnormal amount of TRAP 5b is indicative of susceptibility to such a disorder, wherein the phthalein compound is a compound having the formula:

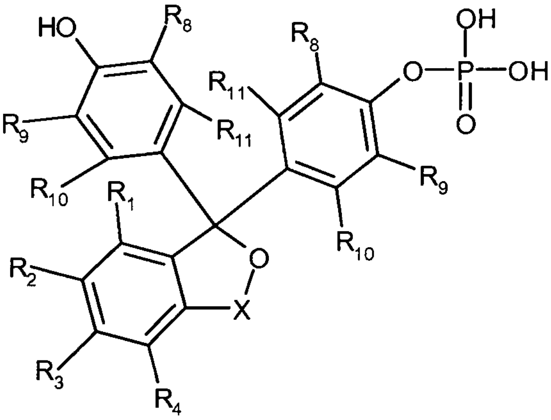

(II)

wherein:

X is CO or $SO_2$;
$R^1$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$;
$R^2$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$;
$R^3$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$;
$R^4$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$;
each $R^8$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$;
each $R^9$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$;
each $R^{10}$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$; and
each $R^{11}$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$;

wherein:

n is 0, 1 or 2;
$R^5$ and $R^6$ are each independently hydrogen or $R^7$; and
$R^7$ is selected from hydrocarbyl, $-(CH_2)_k$-heterocyclyl, $-(CH_2)_k$-C(O)-heterocyclyl, any of which is optionally substituted with 1, 2, 3, 4 or 5 substituents independently selected from halogen, cyano, amino, hydroxy, -COOH and esters thereof (e.g. -C(O)-$C_{1-6}$ alkyl), phosphoramidite, $C_{1-6}$ alkyl and $C_{1-6}$ alkoxy; and k is 0, 1, 2, 3, 4, 5 or 6.

**7.** A method according to any one of claims 2 to 6, comprising (a) binding TRAP present in a sample to an antibody that binds total TRAP (TRAP 5a and TRAP 5b); and (b) obtaining a measure of the amount of TRAP 5b in the sample by assaying the activity of TRAP 5b bound to the antibody using monophosphate ester of a phthalein compound as a substrate, wherein the phthalein compound is a compound having the formula:

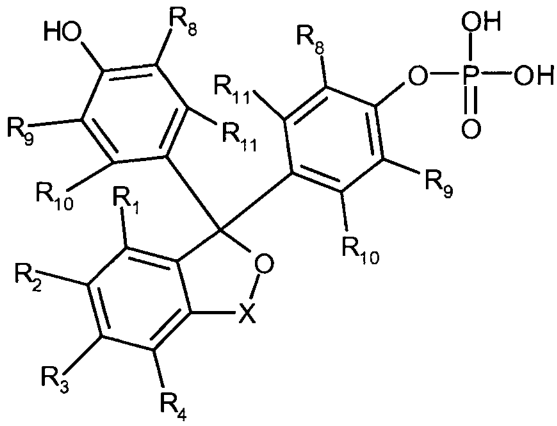

(II)

wherein:

X is CO or $SO_2$;
$R^1$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$;
$R^2$ is selected from hydrogen, halogen, trifluoromethyl, cyano, nitro, $-OR^5$, $-C(O)R^5$,$-C(O)OR^5$, $-OC(O)R^5$, $-S(O)nR^5$, $-N(R^5)R^6$, $-C(O)N(R^5)R^6$ and $R^7$;
$R^3$ is selected from hydrogen, halogen, trifluoromethyl, cyano, nitro, $-OR^5$, $-C(O)R^5$,$-C(O)OR^5$ $-OC(O)R^5$, $-S(O)_nR^5$, $-N(R^5)R^6$, $-C(O)N(R^5)R^6$ and $R^7$;
$R^4$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$;
each $R^8$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$;
each $R^9$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$;
each $R^{10}$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$; and
each $R^{11}$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$;

wherein:

n is 0, 1 or 2;
$R^5$ and $R^6$ are each independently hydrogen or $R^7$; and
$R^7$ is selected from hydrocarbyl, $-(CH_2)_k$-heterocyclyl, $-(CH_2)_k$-C(O)-heterocyclyl, any of which is optionally substituted with 1, 2, 3, 4 or 5 substituents independently selected from halogen, cyano, amino, hydroxy, -COOH and esters thereof (e.g. $-C(O)-C_{1-6}$ alkyl), phosphoramidite, $C_{1-6}$ alkyl and $C_{1-6}$ alkoxy; and k is 0, 1, 2, 3, 4, 5 or 6.

**8.** A method according to any one of claims 2 to 7 wherein the step of assaying the activity of TRAP 5b is performed at pH 6.8 to 7.6, preferably pH 6.8 to 7.4, and most preferably pH 7.

**9.** A method according to any one of claims 2 to 8, wherein the measure of the amount of TRAP 5b is obtained as a measure of the colour of the sample after TRAP 5b activity.

**10.** A method according to any one of claims 1 to 9, wherein the step of assaying the activity of TRAP 5b in a sample comprises the sequential steps of:

(a) incubating the sample with a monophosphate ester of a phthalein compound to allow TRAP 5b to metabolise the substrate;
(b) stopping the metabolism;
(c) optionally adjusting the pH of the sample;
(d) measuring change in a characteristic of the substrate resulting from metabolism by TRAP 5b

**11.** A method according to claims 9 or 10, wherein the colour of the sample is measured using a spectrophotometer.

**12.** A method according to any one of claims 2 to 10, further comprising obtaining an absolute value of the amount of TRAP 5b present in the sample, by comparing the measure of TRAP 5b obtained in any one of claims to a calibrated standard or chart.

**13.** A method according to any one of claims 2 to 11, wherein the step of assaying the activity of TRAP 5b is performed on a solid substrate, preferably a platform, or a multi-well plate or array.

**14.** A method according to claim 13 wherein the platform is automated.

**15.** A method according to claims 13 or 14 wherein the automated platform comprises magnetic beads.

**16.** A kit for carrying out a method according to any one of the preceding claims, comprising a monophosphate ester of a phthalein compound, and an antibody which binds total TRAP, wherein the phthalein compound is a compound having the formula:

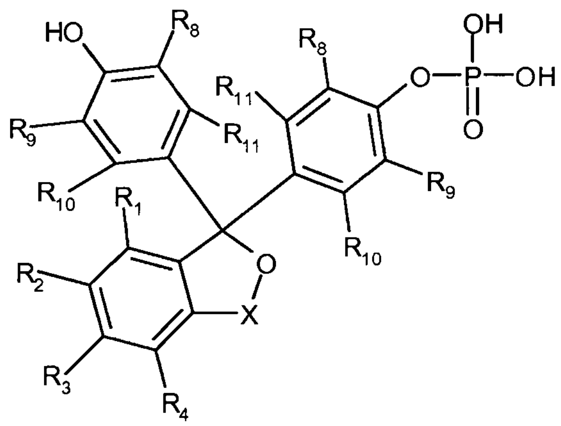

(II)

wherein:

X is CO or $SO_2$;
$R^1$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$;
$R^2$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$;
$R^3$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$;
$R^4$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$;
each $R^8$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$;
each $R^9$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$;
each $R^{10}$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$; and
each $R^{11}$ is selected from hydrogen, halogen, trifluoromethyl, cyano, nitro, $-OR^5$, $-C(O)R^5$,$-C(O)OR^5$, $-OC(O)R^5$, $-S(O)_nR^5$, $-N(R^5)R^6$, $-C(O)N(R^5)R^6$ and $R^7$;

wherein:

n is 0, 1 or 2;
$R^5$ and $R^6$ are each independently hydrogen or $R^7$; and
$R^7$ is selected from hydrocarbyl, $-(CH_2)_k$-heterocyclyl, $-(CH_2)_k-C(O)$-heterocyclyl, any of which is optionally

substituted with 1, 2, 3, 4 or 5 substituents independently selected from halogen, cyano, amino, hydroxy, -COOH and esters thereof (e.g. -C(O)-$C_{1-6}$ alkyl), phosphoramidite, alkyl and $C_{1-6}$ alkoxy; and k is 0, 1, 2, 3, 4, 5 or 6.

**17.** Use of a monophosphate ester of a phthalein compound as an indicator of bone resorption by acting as a specific substrate for TRAP 5b in a sample, wherein the phthalein compound is a compound having the formula:

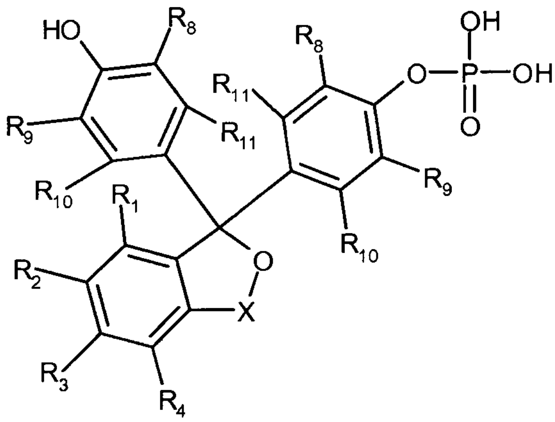

(II)

wherein:

X is CO or $SO_2$;
$R^1$ is selected from hydrogen, halogen, trifluoromethyl, cyano, nitro, -$OR^5$, -C(O)$R^5$,-C(O)O$R^5$, -OC(O)$R^5$, -$S(O)_nR^5$, -N($R^5$)$R^6$, -C(O)N($R^5$)$R^6$ and $R^7$;
$R^2$ is selected from hydrogen, halogen, trifluoromethyl, cyano, nitro, -$OR^5$, -C(O)$R^5$,-C(O)O$R^5$, -OC(O)$R^5$, -$S(O)_nR^5$, -N($R^5$)$R^6$, -C(O)N($R^5$)$R^6$ and $R^7$;
$R^3$ is selected from hydrogen, halogen, trifluoromethyl, cyano, nitro, -$OR^5$, -C(O)$R^5$, - C(O)O$R^5$, -OC(O)$R^5$, -S(O)$R^5$, -N($R^5$)$R^6$, -C(O)N($R^5$)$R^6$ and $R^7$;
$R^4$ is selected from hydrogen, halogen, trifluoromethyl, cyano, nitro, -$OR^5$, -C(O)$R^5$, - C(O)O$R^5$, -OC(O)$R^5$, -$S(O)_nR^5$, -N($R^5$)$R^6$, -C(O)N($R^5$)$R^6$ and $R^7$
each $R^8$ is selected from hydrogen, halogen, trifluoromethyl, cyano, nitro, -$OR^5$, -C(O)$R^5$, - C(O)O$R^5$, -OC(O)$R^5$, -$S(O)_nR^5$, -N($R^5$)$R^6$, -C(O)N($R^5$)$R^6$ and $R^7$;
each $R^9$ is selected from hydrogen, halogen, trifluoromethyl, cyano, nitro, -$OR^5$, -C(O)$R^5$, - C(O)O$R^5$, -OC(O)$R^5$, -$S(O)_nR^5$, -N($R^5$)$R^6$, -C(O)N($R^5$)$R^6$ and $R^7$;
each $R^{10}$ is selected from hydrogen, halogen, trifluoromethyl, cyano, nitro, -$OR^5$, -C(O)$R^5$, - C(O)O$R^5$, -OC(O)$R^5$, -$S(O)_nR^5$, -N($R^5$)$R^6$, -C(O)N($R^5$)$R^6$ and $R^7$; and
each $R^{11}$ is selected from hydrogen, halogen, trifluoromethyl, cyano, nitro, -$OR^5$, -C(O)$R^5$,-C(O)O$R^5$, -OC(O)$R^5$, -$S(O)_nR^5$, -N($R^5$)$R^6$, -C(O)N($R^5$)$R^6$ and $R^7$;

wherein:

n is 0, 1 or 2;
$R^5$ and $R^6$ are each independently hydrogen or $R^7$; and
$R^7$ is selected from hydrocarbyl, -$(CH_2)_k$-heterocyclyl, -$(CH_2)_k$-C(O)-heterocyclyl, any of which is optionally substituted with 1, 2, 3, 4 or 5 substituents independently selected from halogen, cyano, amino, hydroxy, -COOH and esters thereof (e.g. -C(O)-$C_{1-6}$ alkyl), phosphoramidite, $C_{1-6}$ alkyl and $C_{1-6}$ alkoxy; and k is 0, 1, 2, 3, 4, 5 or 6.

**18.** Use according to claim 17 in a method according to any one of claims 1 to 15.

**19.** Use according to claim 17, comprising contacting the monophosphate ester with a sample from a subject and subsequently measuring the colour of the sample.

**20.** A use, method, or kit according to any one of the preceding claims wherein a phthalein compound is a phenolphthalein or a sulphophthalein.

**21.** A use, method or kit according to claim 20, wherein a phthalein compound is a phenolsulphophthalein

**22.** A use, method or kit according to any one of the preceding claims wherein a monophosphate ester of a phthalein compound is selected from the group consisting of phenolphthalein monophosphate, Cresolphthalein monophosphate, Thymolphthalein monophosphate, o-Chlorophenolphthalein monophosphate, Phenol Red monophosphate, o-Cresol Red monophosphate, m-Cresol Purple monophosphate, Bromocresol Purple monophosphate, Bromocresol Green monophosphate, Bromophenol Blue monophosphate, Thymol Blue monophosphate, Bromothymol Blue monophosphate, Xylenol Blue monophosphate, and Chlorophenol Red monophosphate.

## Patentansprüche

**1.** Verwendung eines Monophosphatesters einer Phthaleinverbindung als Substrat zum Erhalten eines spezifischen Maßes der Aktivität und/oder Menge tartratresistenter saurer Phosphatase 5b (TRAP 5b) in einer Probe, wobei die Phthaleinverbindung eine die folgende Formel umfassende Verbindung ist:

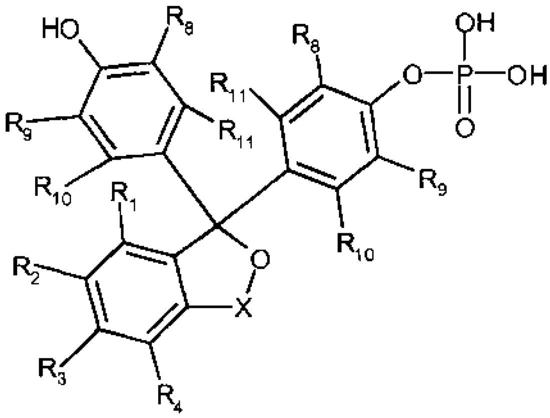

(II)

wobei:

X für CO oder $SO_2$ steht;
$R^1$ aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist;
$R^2$ aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist;
$R^3$ aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist;
$R^4$ aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist;
$R^8$ jeweils aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist;
$R^9$ jeweils aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist;
$R^{10}$ jeweils aus Hydrogen, Halogen, Trifluormethyl, Cyano, Nitro, $-OR^5$, $-C(O)R^5$, $C(O)OR^5$, $-OC(O)R^5$, $-S(O)_nR^5$, $-N(R^5)R^6$, $-C(O)N(R^5)R^6$ und $R^7$ ausgewählt ist; und
$R^{11}$ jeweils aus Hydrogen, Halogen, Trifluormethyl, Cyano, Nitro, $-OR^5$, $-C(O)R^5$, $C(O)OR^5$, $-OC(O)R^5$, $-S(O)_nR^5$, $-N(R^5)R^6$, $-C(O)N(R^5)R^6$ und $R^7$ ausgewählt ist;

wobei:

n für 0, 1 oder 2 steht;
$R^5$ und $R^6$ unabhängig voneinander jeweils Hydrogen oder $R^7$ bedeuten; und
$R^7$ aus Hydrocarbyl, $-(CH_2)_k$-heterocyclyl, $-(CH_2)_k-C(O)$-heterocyclyl ausgewählt ist, von welchen jedes beliebige gegebenenfalls mit 1, 2, 3, 4 oder 5 Substituenten ersetzt wird, voneinander unabhängig aus Halogen,

Cyano, Amino, Hydroxy, -COOH und Estern davon (z. B. -C(O)-$C_{1-6}$-Alkyl), Phosphoramidit, $C_{1-6}$-Alkyl und $C_{1-6}$-Alkoxy ausgewählt; und k für 0, 1, 2, 3, 4, 5 oder 6 steht.

**2.** Verfahren zum Erhalten eines Maßes der TRAP-5b-Menge in einer Probe, umfassend das Untersuchen der TRAP-5b-Aktivität unter Verwendung eines Monophosphatesters einer Phthaleinverbindung als Substrat, wobei die Phthaleinverbindung eine die folgende Formel umfassende Verbindung ist:

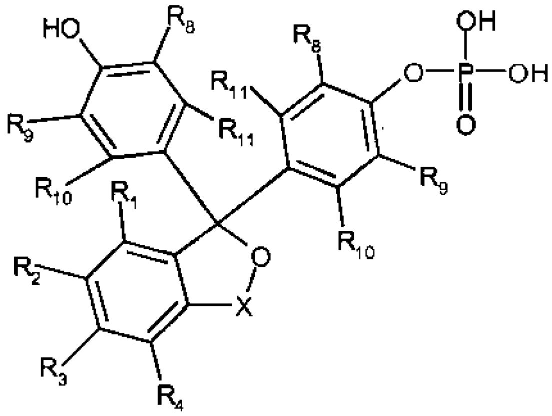

(II)

wobei:

X für CO oder $SO_2$ steht;
$R^1$ aus Hydrogen, Halogen, Trifluormethyl, Cyano, Nitro, -$OR^5$, -C(O)$R^5$, -C(O)$OR^5$, -OC(O)$R^5$, -$S(O)_nR^5$, -N($R^5$)$R^6$, -C(O)N($R^5$)$R^6$ und $R^7$ ausgewählt ist;
$R^2$ aus Hydrogen, Halogen, Trifluormethyl, Cyano, Nitro, -$OR^5$, -C(O)$R^5$, -C(O)$OR^5$, -OC(O)$R^5$, -$S(O)_nR^5$, -N($R^5$)$R^6$, -C(O)N($R^5$)$R^6$ und $R^7$ ausgewählt ist;
$R^3$ aus Hydrogen, Halogen, Trifluormethyl, Cyano, Nitro, -$OR^5$, -C(O)$R^5$, -C(O)$OR^5$, -OC(O)$R^5$, -$S(O)_nR^5$, -N($R^5$)$R^6$, -C(O)N($R^5$)$R^6$ und $R^7$ ausgewählt ist;
$R^4$ aus Hydrogen, Halogen, Trifluormethyl, Cyano, Nitro, -$OR^5$, -C(O)$R^5$, -C(O)$OR^5$, -OC(O)$R^5$, -$S(O)_nR^5$, -N($R^5$)$R^6$, -C(O)N($R^5$)$R^6$ und $R^7$ ausgewählt ist;
$R^8$ jeweils aus Hydrogen, Halogen, Trifluormethyl, Cyano, Nitro, -$OR^5$, -C(O)$R^5$, -C(O)$OR^5$, -OC(O)$R^5$, -$S(O)_nR^5$, -N($R^5$)$R^6$, -C(O)N($R^5$)$R^6$ und $R^7$ ausgewählt ist;
$R^9$ jeweils aus Hydrogen, Halogen, Trifluormethyl, Cyano, Nitro, -$OR^5$, -C(O)$R^5$, -C(O)$OR^5$, -OC(O)$R^5$, -$S(O)_nR^5$, -N($R^5$)$R^6$, -C(O)N($R^5$)$R^6$ und $R^7$ ausgewählt ist;
$R^{10}$ jeweils aus Hydrogen, Halogen, Trifluormethyl, Cyano, Nitro, -$OR^5$, -C(O)$R^5$, -C(O)$OR^5$, -OC(O)$R^5$, -$S(O)_nR^5$, -N($R^5$)$R^6$, -C(O)N($R^5$)$R^6$ und $R^7$ ausgewählt ist; und
$R^{11}$ jeweils aus Hydrogen, Halogen, Trifluormethyl, Cyano, Nitro, -$OR^5$, -C(O)$R^5$, -C(O)$OR^5$, -OC(O)$R^5$, -$S(O)_nR^5$, -N($R^5$)$R^6$, -C(O)N($R^5$)$R^6$ und $R^7$ ausgewählt ist;

wobei:

n für 0, 1 oder 2 steht;
$R^5$ und $R^6$ unabhängig voneinander jeweils Hydrogen oder $R^7$ bedeuten; und
$R^7$ aus Hydrocarbyl, -$(CH_2)_k$-heterocyclyl, -$(CH_2)_k$-C(O)-heterocyclyl ausgewählt ist, von welchen jedes beliebige gegebenenfalls mit 1, 2, 3, 4 oder 5 Substituenten ersetzt wird, voneinander unabhängig aus Halogen, Cyano, Amino, Hydroxy, -COOH und Estern davon (z. B. -C(O)-$C_{1-6}$-Alkyl), Phosphoramidit, $C_{1-6}$-Alkyl und $C_{1-6}$-Alkoxy ausgewählt; und k für 0, 1, 2, 3, 4, 5 oder 6 steht.

**3.** Verfahren zum Messen der Knochenresorptionsrate bei einem Individuum, umfassend das Erhalten eines Maßes der TRAP-5b-Menge in einer Probe von einem Individuum durch Untersuchen der TRAP-5b-Aktivität unter Verwendung eines Monophosphatesters einer Phthaleinverbindung als Substrat, wobei die in der Probe vorliegende TRAP-5b-Menge ein Anhaltspunkt für die Knochenabsorptionsrate ist, wobei die Phthaleinverbindung eine die folgende Formel umfassende Verbindung ist:

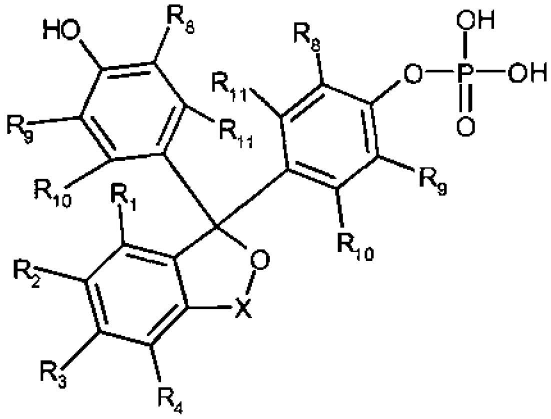

(II)

wobei:

X für CO oder $SO_2$ steht;
$R^1$ aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist;
$R^2$ aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist;
$R^3$ aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist;
$R^4$ aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist;
$R^8$ jeweils aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist;
$R^9$ jeweils aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist;
$R^{10}$ jeweils aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist; und
$R^{11}$ jeweils aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist;

wobei:

n für 0, 1 oder 2 steht;
$R^5$ und $R^6$ unabhängig voneinander jeweils Hydrogen oder $R^7$ bedeuten; und
$R^7$ aus Hydrocarbyl, $-(CH_2)_k$-heterocyclyl, $-(CH_2)_k$-C(O)-heterocyclyl ausgewählt ist, von welchen jedes beliebige gegebenenfalls mit 1, 2, 3, 4 oder 5 Substituenten ersetzt ist, voneinander unabhängig aus Halogen, Cyano, Amino, Hydroxy, -COOH und Estern davon (z. B. $-C(O)-C_{1-6}$-Alkyl), Phosphoramidit, $C_{1-6}$-Alkyl und $C_{1-6}$-Alkoxy ausgewählt; und k für 0, 1, 2, 3, 4, 5 oder 6 steht.

**4.** Verfahren zum Diagnostizieren der Prädisposition für Störungen, welche mit einer Veränderung der Knochenresorptionsrate bei einem Individuum verbunden sind, umfassend das Erhalten eines Maßes der TRAP-5b-Menge, die in einer Probe des Individuums vorhanden ist, durch Untersuchen der TRAP-5b-Aktivität unter Verwendung eines Monophosphatesters einer Phthaleinverbindung als Substrat, wobei eine ungewöhnliche TRAP-5b-Menge darauf hindeutet, dass das Individuum die Prädisposition besitzt, wobei die Phthaleinverbindung eine die folgende Formel umfassende Verbindung ist:

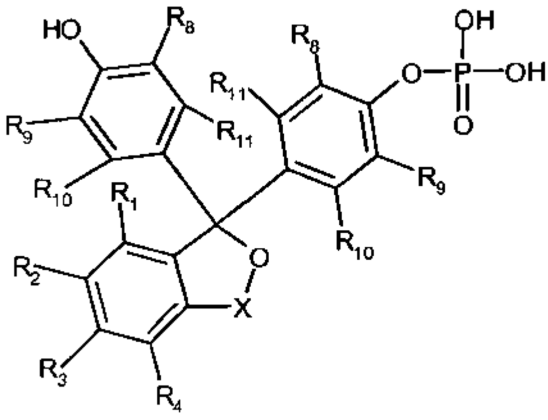

(II)

wobei:

X für CO oder $SO_2$ steht;
$R^1$ aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist;
$R^2$ aus Hydrogen, Halogen, Trifluormethyl, Cyano, Nitro, $-OR^5$, $-C(O)R^5$, $-C(O)OR^5$ , $-OC(O)R^5$ , $-S(O)_nR^5$, $-N(R^5)R^6$, $-C(O)N(R^5)R^6$ und $R^7$ ausgewählt ist;
$R^3$ aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist;
$R^4$ aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist;
$R^8$ jeweils aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist;
$R^9$ jeweils aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist;
$R^{10}$ jeweils aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist; und
$R^{11}$ jeweils aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist;

wobei:

n für 0, 1 oder 2 steht;
$R^5$ und $R^6$ unabhängig voneinander Hydrogen oder $R^7$ bedeuten; und
$R^7$ aus Hydrocarbyl, $-(CH_2)_k$-heterocyclyl, $-(CH_2)_k-C(O)$-heterocyclyl ausgewählt ist, von denen jedes beliebige gegebenenfalls mit 1, 2, 3, 4 oder 5 Substituenten ersetzt ist, voneinander unabhängig aus Halogen, Cyano, Amino, Hydroxy, -COOH und Estern davon (z. B. $-C(O)-C_{1-6}$-Alkyl), Phosphoramidit, $C_{1-6}$-Alkyl und $C_{1-6}$-Alkoxy ausgewählt; und k für 0, 1, 2, 3, 4, 5 oder 6 steht.

**5.** Verfahren zum Überwachen der Wirkung eines Arzneimittels zur Prävention oder Behandlung einer Störung, welche mit einer Veränderung der Knochenresorptionsrate bei einem das Arzneimittel einnehmenden Individuum verbunden ist, umfassend (a) das Erhalten eines Maßes der TRAP-5b-Menge, die in einer Probe des Individuums vorhanden ist, durch Untersuchen der TRAP-5b-Aktivität unter Verwendung eines Monophosphatesters einer Phthaleinverbindung als Substrat; und (b) Vergleichen der TRAP-5b-Menge in der Probe mit der TRAP-5b-Menge in einer Kontrollprobe, wobei die Phthaleinverbindung eine die folgende Formel umfassende Verbindung ist:

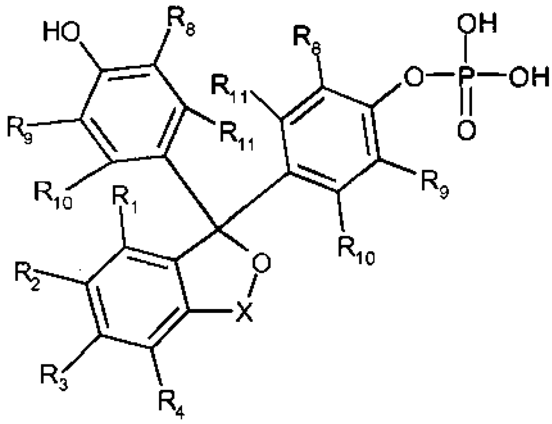

(II)

wobei:

X für CO oder $SO_2$ steht;
$R^1$ aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist;
$R^2$ aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist;
$R^3$ aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist;
$R^4$ aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist;
$R^8$ jeweils aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist;
$R^9$ jeweils aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist;
$R^{10}$ jeweils aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist; und
$R^{11}$ jeweils aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist;

wobei:

n für 0, 1 oder 2 steht;
$R^5$ und $R^6$ unabhängig voneinander jeweils Hydrogen oder $R^7$ bedeuten; und
$R^7$ aus Hydrocarbyl, $-(CH_2)_k$-heterocyclyl, $-(CH_2)_k$-C(O)-heterocyclyl ausgewählt ist, von welchen jedes beliebige gegebenenfalls mit 1, 2, 3, 4 oder 5 Substituenten ersetzt wird, voneinander unabhängig aus Halogen, Cyan, Amino, Hydroxy, -COOH und Estern davon (z. B. -C(O)-$C_{1-6}$-Alkyl), Phosphoramidit, $C_{1-6}$-Alkyl und $C_{1-6}$-Alkoxy ausgewählt; und k für 0, 1, 2, 3, 4, 5 oder 6 steht.

**6.** Verfahren zum Screenen nach der Prädisposition eines Individuums für eine Störung, welche mit einer Veränderung der Knochenresorptionsrate verbunden ist, umfassend das Erhalten eines Maßes der TRAP-5b-Menge, die in einer Probe des Individuums vorhanden ist, durch Untersuchen der TRAP-5b-Aktivität unter Verwendung eines Monophosphatesters einer Phthaleinverbindung als Substrat, wobei eine ungewöhnliche TRAP-5b-Menge auf die Prädisposition für eine solche Störung hindeutet, wobei die Phthaleinverbindung eine die folgende Formel umfassende Verbindung ist:

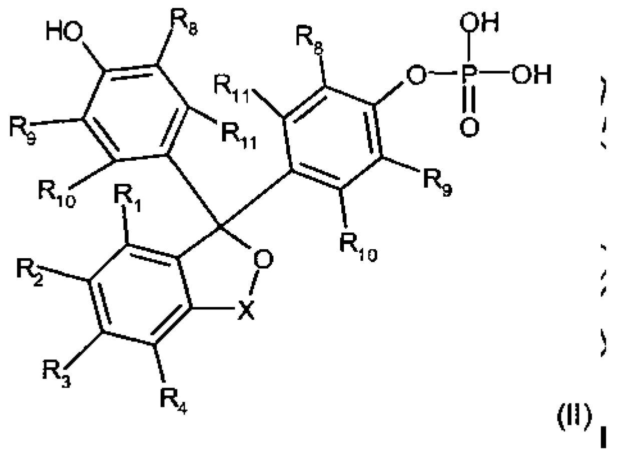

(II)

wobei:

X für CO oder $SO_2$ steht;
$R^1$ aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist;
$R^2$ aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist;
$R^3$ aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist;
$R^4$ aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist;
$R^8$ jeweils aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist;
$R^9$ jeweils aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist;
$R^{10}$ jeweils aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist; und
$R^{11}$ jeweils aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist;

wobei:

n für 0, 1 oder 2 steht;
$R^5$ und $R^6$ unabhängig voneinander Hydrogen oder $R^7$ bedeuten; und
$R^7$ aus Hydrocarbyl, $-(CH_2)_k$-heterocyclyl, $-(CH_2)_k$-C(O)-heterocyclyl ausgewählt ist, von denen jedes beliebige gegebenenfalls mit 1, 2, 3, 4 oder 5 Substituenten ersetzt wird, voneinander unabhängig aus Halogen, Cyano, Amino, Hydroxy, -COOH und Estern davon (z. B. $-C(O)-C_{1-6}$-Alkyl), Phosphoramidit, $C_{1-6}$-Alkyl und $C_{1-6}$-Alkoxy ausgewählt; und k für 0, 1, 2, 3, 4, 5 oder 6 steht.

7. Verfahren nach einem der Ansprüche 2 bis 6, umfassend (a) das Binden der in einer Probe vorhandenen TRAP an einen Antikörper, welcher die Gesamt-TRAP (TRAP 5a und TRAP 5b) bindet; und (b) das Erhalten eines Maßes der TRAP-5b-Menge in der Probe durch Untersuchen der Aktivität der an den Antikörper gebundenen TRAP 5b unter Verwendung eines Monophosphatesters einer Phthaleinverbindung als Substrat, wobei die Phthaleinverbindung eine die folgende Formel umfassende Verbindung ist:

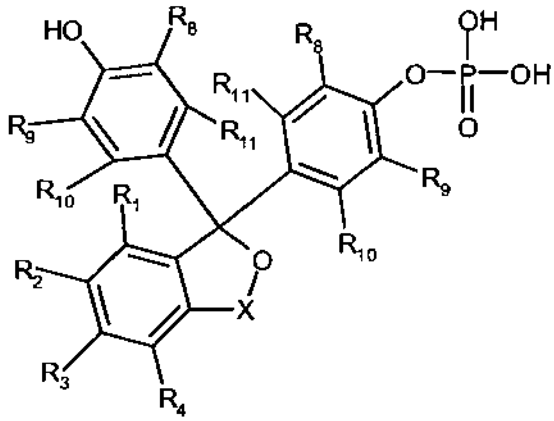

(II)

wobei:

X für CO oder $SO_2$ steht;
$R^1$ aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist;
$R^2$ aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist;
$R^3$ aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist;
$R^4$ aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist;
$R^8$ jeweils aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist;
$R^9$ jeweils aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist;
$R^{10}$ jeweils aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist; und
$R^{11}$ jeweils aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist;

wobei:

n für 0, 1 oder 2 steht;
$R^5$ und $R^6$ unabhängig voneinander Hydrogen oder $R^7$ bedeuten; und
$R^7$ aus Hydrocarbyl, $-(CH_2)_k$-heterocyclyl, $-(CH_2)_k-C(O)$-heterocyclyl ausgewählt ist, von denen jedes beliebige gegebenenfalls mit 1, 2, 3, 4 oder 5 Substituenten ersetzt wird, voneinander unabhängig aus Halogen, Cyano, Amino, Hydroxy, -COOH und Estern davon (z. B. $-C(O)-C_{1-6}$-Alkyl), Phosphoramidit, $C_{1-6}$-Alkyl und $C_{1-6}$-Alkoxy ausgewählt; und k für 0, 1, 2, 3, 4, 5 oder 6 steht.

**8.** Verfahren nach einem der Ansprüche 2 bis 7, wobei der Schritt des Untersuchens der TRAP-5b-Aktivität bei einem pH-Wert von 6,8 bis 7,6, bevorzugt bei einem pH-Wert von 6,8 bis 7,4, am bevorzugtesten bei einem pH-Wert von 7 ausgeführt wird.

**9.** Verfahren nach einem der Ansprüche 2 bis 8, wobei das Maß der TRAP-5b-Menge als ein Maß der Farbe der Probe nach der TRAP-5b-Aktivität erhalten wird.

**10.** Verfahren nach einem der Ansprüche 1 bis 9, wobei der Schritt des Untersuchens der TRAP-5b-Aktivität in einer Probe folgende Ablaufschritte umfasst:

(a) Inkubieren der Probe mit einem Monophosphatester einer Phthaleinverbindung, um TRAP 5b die Metabolisierung des Substrats zu erlauben;

(b) Stoppen des Metabolismus;
(c) gegebenenfalls Einstellen des pH-Wertes der Probe;
(d) Messen von Veränderungen in einer Eigenschaft des Substrats infolge des Metabolismus durch TRAP 5b

**11.** Verfahren nach Anspruch 9 oder 10, wobei die Farbe der Probe unter Verwendung eines Spektrometers gemessen wird.

**12.** Verfahren nach einem der Ansprüche 2 bis 10, ferner umfassend das Erhalten eines Absolutwertes der in der Probe vorhandenen TRAP-5b-Menge durch Vergleichen des nach einem der Ansprüche erhaltenen TRAP-5b-Maßes mit einem kalibrierten Standard oder Diagramm.

**13.** Verfahren nach einem der Ansprüche 2 bis 11, wobei der Schritt des Untersuchens der TRAP-5b-Aktivität auf einem harten Substrat, vorzugsweise einer Plattform oder einer Multiwellplatte oder einem Array ausgeführt wird.

**14.** Verfahren nach Anspruch 13, wobei die Plattform automatisiert ist.

**15.** Verfahren nach Anspruch 13 oder 14, wobei die automatisierte Plattform magnetische Kugeln umfasst.

**16.** Set zum Durchführen eines Verfahrens nach einem der vorhergehenden Ansprüche, umfassend ein Monophosphatester einer Phthaleinverbindung und einen die Gesamt-TRAP bindenden Antikörper, wobei die Phthaleinverbindung eine die folgende Formel umfassende Verbindung ist:

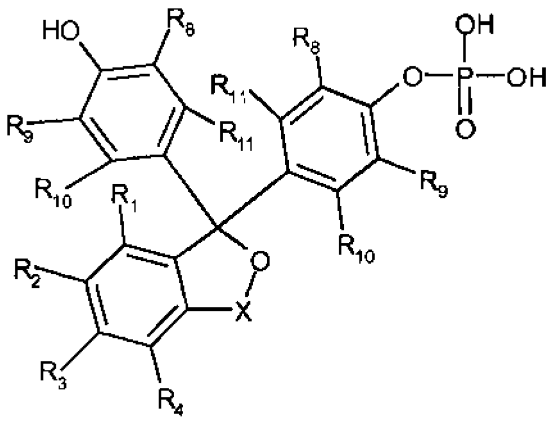

**(II)**

wobei:

X für CO oder $SO_2$ steht;
$R^1$ aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist;
$R^2$ aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist;
$R^3$ aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist;
$R^4$ aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist;
$R^8$ jeweils aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist;
$R^9$ jeweils aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist;
$R^{10}$ jeweils aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist; und
$R^{11}$ jeweils aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist;

wobei:

n für 0, 1 oder 2 steht;
$R^5$ und $R^6$ unabhängig voneinander Hydrogen oder $R^7$ bedeuten; und
$R^7$ aus Hydrocarbyl, $-(CH_2)_k$-heterocyclyl, $-(CH_2)_k$-C(O)-heterocyclyl ausgewählt ist, von welchen jedes beliebige gegebenenfalls mit 1, 2, 3, 4 oder 5 Substituenten ersetzt wird, voneinander unabhängig aus Halogen, Cyano, Amino, Hydroxy, -COOH und Estern davon (z. B. -C(O)-$C_1$-$_6$-Alkyl), Phosphoramidit, $C_1$-$_6$-Alkyl und $C_1$-$_6$-Alkoxy ausgewählt; und k für 0, 1, 2, 3, 4, 5 oder 6 steht.

**17.** Verwendung eines Monophosphatesters einer Phthaleinverbindung als Indikator für die Knochenresorption, indem er als ein spezifisches Substrat für TRAP 5b in einer Probe fungiert, wobei die Phthaleinverbindung eine die folgende Formel umfassende Verbindung ist:

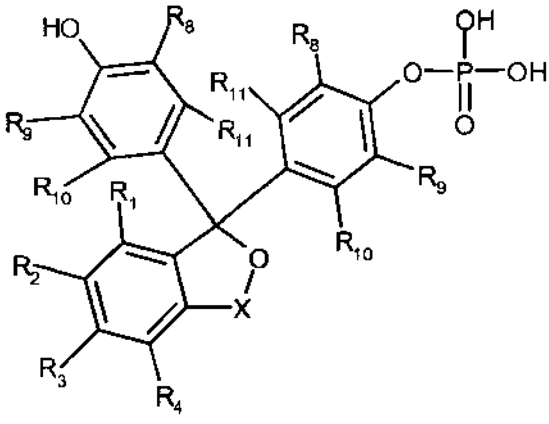

(II)

wobei:

X für CO oder $SO_2$ steht;
$R^1$ aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist;
$R^2$ aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist;
$R^3$ aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist;
$R^4$ aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist;
$R^8$ jeweils aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist;
$R^9$ jeweils aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist;
$R^{10}$ jeweils aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist; und
$R^{11}$ jeweils aus Hydrogen, Halogen, Trifluormethyl, Cyano, Nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ und $R^7$ ausgewählt ist;

wobei:

n für 0, 1 oder 2 steht;
$R^5$ und $R^6$ unabhängig voneinander Hydrogen oder $R^7$ bedeuten; und
$R^7$ aus Hydrocarbyl, $-(CH_2)$k-heterocyclyl, $-(CH_2)$k-C(O)-heterocyclyl ausgewählt ist, von welchen jedes beliebige gegebenenfalls mit 1, 2, 3, 4 oder 5 Substituenten ersetzt wird, voneinander unabhängig aus Halogen, Cyano, Amino, Hydroxy, -COOH und Estern davon (z. B. -C(O)-$C_{1-6}$-Alkyl), Phosphoramidit, $C_{1-6}$-Alkyl und $C_{1-6}$-Alkoxy ausgewählt; und k für 0, 1, 2, 3, 4, 5 oder 6 steht.

**18.** Verwendung nach Anspruch 17 in einem Verfahren nach einem der Ansprüche 1 bis 15.

**19.** Verwendung nach Anspruch 17, umfassend das Inkontaktbringen des Monophosphatesters mit einer Probe von einem Individuum und das darauffolgende Messen der Farbe der Probe.

**20.** Verwendung, Verfahren oder Set nach einem der vorhergehenden Ansprüche, wobei eine Phthaleinverbindung ein Phenolphthalein oder ein Sulfophthalein ist.

**21.** Verwendung, Verfahren oder Set nach Anspruch 20, wobei eine Phthaleinverbindung ein Phenolsulfophthalein ist.

**22.** Verwendung, Verfahren oder Set nach einem der vorhergehenden Ansprüche, wobei ein Monophosphatester einer Phthaleinverbindung aus der Gruppe ausgewählt ist, welche aus Folgenden besteht: Phenolphthalein-monophosphat, Cresolphthalein-monophosphat, Thymolphthalein-monophosphat, o-Chlorphenolphthalein-monophosphat, Phenolrot-monophosphat, o-Cresolrot-monophosphat, m-Cresolpurpur-monophosphat, Bromcresolpurpur-monophosphat, Bromcresolgrün-monophosphat, Bromphenolblau-monophosphat, Thymolblau-monophosphat, Bromthymolblau-monophosphat, Xylenolblau-monophosphat und Chlorphenolrot-monophosphat.

## Revendications

**1.** Utilisation d'un ester monophosphate d'un composé de phtaléine en tant que substrat pour obtenir une mesure de l'activité et/ou de la quantité de phosphatase acide 5b résistant au tartrate (TRAP 5b) dans un échantillon, ledit composé de phtaléine étant un composé ayant la formule :

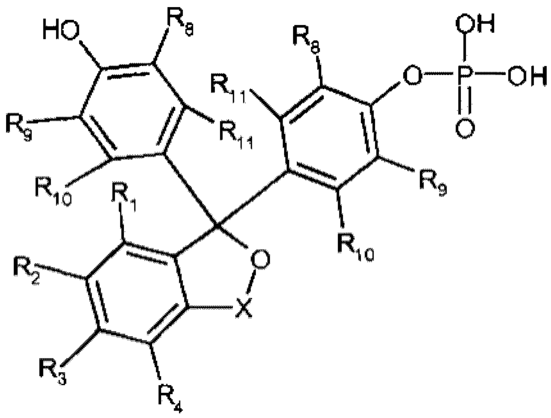

**(II)**

dans laquelle :

X représente un groupe CO ou $SO_2$;
$R^1$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ et $R^7$;
$R^2$ est choisi parmi un atome d'hydrogène, d'halogène, un groupe trifluorométhyle, cyano, nitro, $-OR^5$, $-C(O)R^5$,-$C(O)OR^5$, $-OC(O)R^5$, $-S(O)_nR^5$, $-N(R^5)R^6$, $-C(O)N(R^5)R^6$ et $R^7$;
$R^3$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ et $R^7$;
$R^4$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ et $R^7$;
chaque groupe $R^8$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$,$-C(O)N(R^5)R^6$ et $R^7$;
chaque groupe $R^9$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$,$-C(O)N(R^5)R^6$ et $R^7$;
chaque groupe $R^{10}$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$,$-C(O)N(R^5)R^6$ et $R^7$; et
chaque groupe $R^{11}$ est choisi parmi un atome d'hydrogène, d'halogène, un groupe trifluorométhyle, cyano,

nitro, $-OR^5$, $-C(O)R^5$, $-C(O)OR^5$, $-OC(O)R^5$, $-S(O)_nR^5$, $-N(R^5)R^6$,$-C(O)N(R^5)R^6$ et $R^7$;

dans lesquels :

n est égal à 0, 1 ou 2 ;
$R^5$ et $R^6$ représentent chacun indépendamment un atome d'hydrogène ou un groupe $R^7$; et
$R^7$ est choisi parmi un groupe hydrocarbyle, $-(CH_2)_k$-hétérocyclyle, $-(CH_2)_k$-C(O)-hétérocyclyle, l'un quelconque desquels étant éventuellement substitué par 1, 2, 3, 4 ou 5 substituants indépendamment choisis parmi un atome d'halogène, un groupe cyano, amino, hydroxy, -COOH et des groupes esters de ceux-ci (par ex. -C(O)-($C_1$ à $C_6$)alkyle), un groupe phosphoramidite, alkyle $C_1$ à $C_6$ et alcoxy en $C_1$ à $C_6$; et k est égal à 0, 1, 2, 3, 4, 5 ou 6.

**2.** Méthode permettant d'obtenir une mesure de la quantité de TRAP 5b dans un échantillon, comprenant l'analyse de l'activité de TRAP 5b à l'aide d'un ester monophosphate d'un composé de phtaléine en tant que substrat, ledit composé de phtaléine étant un composé ayant la formule :

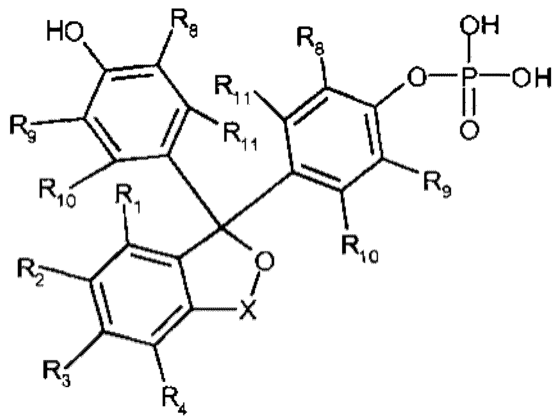

**(II)**

dans laquelle :

X représente un groupe CO ou $SO_2$;
$R^1$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ et $R^7$;
$R^2$ est choisi parmi un atome d'hydrogène, d'halogène, un groupe trifluorométhyle, cyano, nitro, $-OR^5$, $-C(O)R^5$,-$C(O)OR^5$, $-OC(O)R^5$, $-S(O)_nR^5$, $-N(R^5)R^6$, $-C(O)N(R^5)R^6$ et $R^7$;
$R^3$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ et $R^7$;
$R^4$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ et $R^7$;
chaque groupe $R^8$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$,$-C(O)N(R^5)R^6$ et $R^7$;
chaque groupe $R^9$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$,$-C(O)N(R^5)R^6$ et $R^7$;
chaque groupe $R^{10}$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$,$-C(O)N(R^5)R^6$ et $R^7$; et
chaque groupe $R^{11}$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$,$-C(O)N(R^5)R^6$ et $R^7$;

dans lesquels :

n est égal à 0, 1 ou 2 ;
$R^5$ et $R^6$ représentent chacun indépendamment un atome d'hydrogène ou un groupe $R^7$; et
$R^7$ est choisi parmi un groupe hydrocarbyle, $-(CH_2)_k$-hétérocyclyle, $-(CH_2)_k$-C(O)-hétérocyclyle, l'un quelconque desquels étant éventuellement substitué par 1, 2, 3, 4 ou 5 substituants indépendamment choisis parmi un atome d'halogène, un groupe cyano, amino, hydroxy, -COOH et des groupes esters de ceux-ci (par ex. -C(O)-($C_1$

à $C_6$)alkyle), un groupe phosphoramidite, alkyle en $C_1$ à $C_6$ et alcoxy en $C_1$ à $C_6$; et k est égal à 0, 1, 2, 3, 4, 5 ou 6.

**3.** Méthode permettant de mesurer la vitesse de résorption osseuse chez un sujet, comprenant l'obtention d'une mesure de la quantité de TRAP 5b dans un échantillon provenant d'un sujet en analysant l'activité de TRAP 5b à l'aide d'un ester monophosphate d'un composé de phtaléine en tant que substrat, ladite quantité de TRAP 5b présente dans l'échantillon étant représentative de la vitesse de résorption osseuse, ledit composé de phtaléine étant un composé ayant la formule :

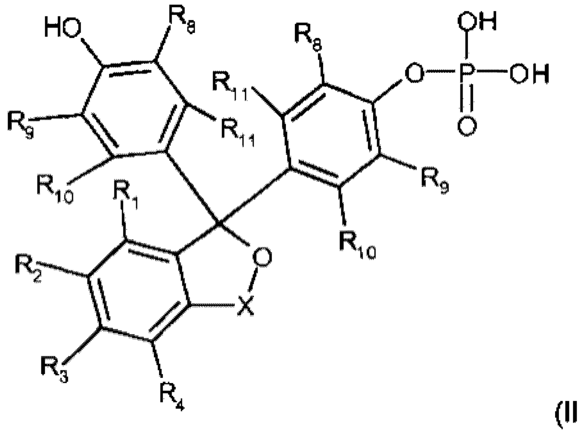

**(II)**

dans laquelle :

X représente un groupe CO ou $SO_2$;
$R^1$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ et $R^7$;
$R^2$ est choisi parmi un atome d'hydrogène, d'halogène, un groupe trifluorométhyle, cyano, nitro, $-OR^5$, $-C(O)R^5$,-$C(O)OR^5$, $-OC(O)R^5$, $-S(O)_nR^5$, $-N(R^5)R^6$, $-C(O)N(R^5)R^6$ et $R^7$;
$R^3$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ et $R^7$;
$R^4$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ et $R^7$;
chaque groupe $R^8$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$,$-C(O)N(R^5)R^6$ et $R^7$;
chaque groupe $R^9$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$,$-C(O)N(R^5)R^6$ et $R^7$;
chaque groupe $R^{10}$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$, - $C(O)N(R^5)R^6$ et $R^7$; et
chaque groupe $R^{11}$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$,$-C(O)N(R^5)R^6$ et $R^7$;

dans lesquels :

n est égal à 0, 1 ou 2 ;
$R^5$ et $R^6$ représentent chacun indépendamment un atome d'hydrogène ou un groupe $R^7$; et
$R^7$ est choisi parmi un groupe hydrocarbyle, $-(CH_2)_k$-hétérocyclyle, $-(CH_2)_k$-C(O)-hétérocyclyle, l'un quelconque desquels étant éventuellement substitué par 1, 2, 3, 4 ou 5 substituants indépendamment choisis parmi un atome d'halogène, un groupe cyano, amino, hydroxy, -COOH et des groupes esters de ceux-ci (par ex. -C(O)-($C_1$ à $C_6$)alkyle), un groupe phosphoramidite, alkyle en $C_1$ à $C_6$ et alcoxy en $C_1$ à $C_6$; et k est égal à 0, 1, 2, 3, 4, 5 ou 6.

**4.** Méthode permettant de diagnostiquer la prédisposition à des troubles associés à un changement de vitesse de résorption osseuse chez un sujet, comprenant l'obtention d'une mesure de la quantité de TRAP 5b présente dans un échantillon du sujet en analysant l'activité de la TRAP 5b à l'aide d'un ester monophosphate d'un composé de phtaléine en tant que substrat, une quantité anormale de TRAP 5b indiquant que le sujet est atteint du trouble, ledit composé de phtaléine étant un composé ayant la formule :

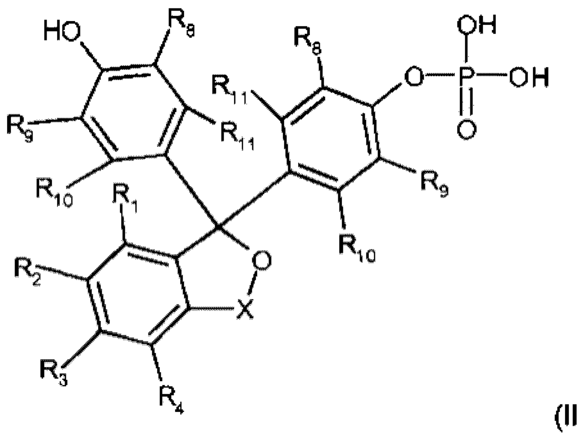

(II)

dans laquelle :

X représente un groupe CO ou $SO_2$;
$R^1$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ et $R^7$;
$R^2$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ et $R^7$;
$R^3$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ et $R^7$;
$R^4$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ et $R^7$;
chaque groupe $R^8$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$,$-C(O)N(R^5)R^6$ et $R^7$;
chaque groupe $R^9$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$,$-C(O)N(R^5)R^6$ et $R^7$;
chaque groupe $R^{10}$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$,$-C(O)N(R^5)R^6$ et $R^7$; et
chaque groupe $R^{11}$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$,$-C(O)N(R^5)R^6$ et $R^7$;

dans lesquels :

n est égal à 0, 1 ou 2 ;
$R^5$ et $R^6$ représentent chacun indépendamment un atome d'hydrogène ou un groupe $R^7$; et
$R^7$ est choisi parmi un groupe hydrocarbyle, $-(CH_2)_k$-hétérocyclyle, $-(CH_2)_k$-C(O)-hétérocyclyle, l'un quelconque desquels étant éventuellement substitué par 1, 2, 3, 4 ou 5 substituants indépendamment choisis parmi un atome d'halogène, un groupe cyano, amino, hydroxy, -COOH et des groupes esters de ceux-ci (par ex. -C(O)-($C_1$ à $C_6$)alkyle), un groupe phosphoramidite, alkyle en $C_1$ à $C_6$ et alcoxy en $C_1$ à $C_6$; et k est égal à 0, 1, 2, 3, 4, 5 ou 6.

**5.** Méthode permettant de surveiller l'effet d'un médicament destiné à la prévention ou au traitement d'un trouble associé à un changement de vitesse de résorption osseuse chez un sujet prenant le médicament, comprenant (a) l'obtention d'une mesure de la quantité de TRAP 5b présente dans un échantillon du sujet en analysant l'activité de la TRAP 5b à l'aide d'un ester monophosphate d'un composé de phtaléine en tant que substrat; et (b) la comparaison de la quantité de TRAP 5b dans l'échantillon la quantité de TRAP 5b dans un échantillon témoin, ledit composé de phtaléine étant un composé ayant la formule :

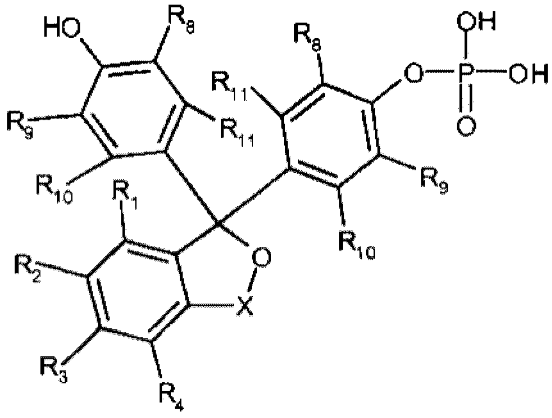

(II)

dans laquelle :

X représente un groupe CO ou $SO_2$;
$R^1$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ et $R^7$;
$R^2$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ et $R^7$;
$R^3$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ et $R^7$;
$R^4$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ et $R^7$;
chaque groupe $R^8$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$,$-C(O)N(R^5)R^6$ et $R^7$;
chaque groupe $R^9$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$,$-C(O)N(R^5)R^6$ et $R^7$;
chaque groupe $R^{10}$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$,$-C(O)N(R^5)R^6$ et $R^7$; et
chaque groupe $R^{11}$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$,$-C(O)N(R^5)R^6$ et $R^7$;

dans lesquels :

n est égal à 0, 1 ou 2 ;
$R^5$ et $R^6$ représentent chacun indépendamment un atome d'hydrogène ou un groupe $R^7$; et
$R^7$ est choisi parmi un groupe hydrocarbyle, $-(CH_2)_k$-hétérocyclyle, $-(CH_2)_k$-C(O)-hétérocyclyle, l'un quelconque desquels étant éventuellement substitué par 1, 2, 3, 4 ou 5 substituants indépendamment choisis parmi un atome d'halogène, un groupe cyano, amino, hydroxy, -COOH et des groupes esters de ceux-ci (par ex. -C(O)-($C_1$ à $C_6$)alkyle), un groupe phosphoramidite, alkyle en $C_1$ à $C_6$ et alcoxy en $C_1$ à $C_6$; et k est égal à 0, 1, 2, 3, 4, 5 ou 6.

**6.** Méthode de dépistage pour déterminer la prédisposition d'un sujet à un trouble associé à un changement de vitesse de résorption osseuse, comprenant l'obtention d'une mesure de la quantité de TRAP 5b présente dans un échantillon du sujet en analysant l'activité de la TRAP 5b à l'aide d'un ester monophosphate d'un composé de phtaléine en tant que substrat, une quantité anormale de TRAP 5b indiquant la prédisposition à un tel trouble, ledit composé de phtaléine étant un composé ayant la formule :

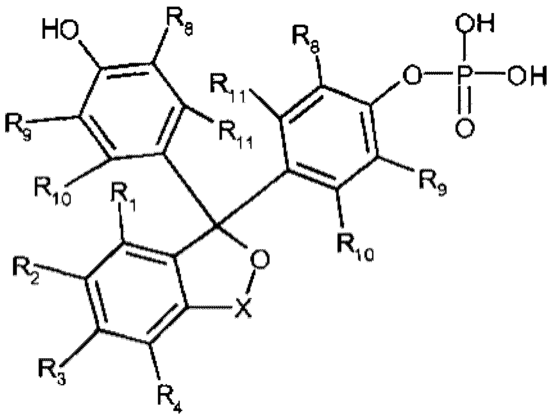

(II)

dans laquelle :

X représente un groupe CO ou $SO_2$;
$R^1$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ et $R^7$;
$R^2$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, -N(Rs)R6, $-C(O)N(R^5)R^6$ et $R^7$;
$R^3$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ et $R^7$;
$R^4$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ et $R^7$;
chaque groupe $R^8$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$, $- C(O)N(R^5)R^6$ et $R^7$;
chaque groupe $R^9$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$,$-C(O)N(R^5)R^6$ et $R^7$;
chaque groupe $R^{10}$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$,$-C(O)N(R^5)R^6$ et $R^7$; et
chaque groupe $R^{11}$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$,$-C(O)N(R^5)R^6$ et $R^7$;

dans lesquels :

n est égal à 0, 1 ou 2 ;
$R^5$ et $R^6$ représentent chacun indépendamment un atome d'hydrogène ou un groupe $R^7$; et
$R^7$ est choisi parmi un groupe hydrocarbyle, $-(CH_2)_k$-hétérocyclyle, $-(CH_2)_k$-C(O)-hétérocyclyle, l'un quelconque desquels étant éventuellement substitué par 1, 2, 3, 4 ou 5 substituants indépendamment choisis parmi un atome d'halogène, un groupe cyano, amino, hydroxy, -COOH et des groupes esters de ceux-ci (par ex. -C(O)-($C_1$ à $C_6$)alkyle), un groupe phosphoramidite, alkyle en $C_1$ à $C_6$ et alcoxy en $C_1$ à $C_6$; et k est égal à 0, 1, 2, 3, 4, 5 ou 6.

**7.** Méthode selon l'une quelconque des revendications 2 à 6, comprenant (a) la liaison de la TRAP présente dans un échantillon à un anticorps qui fixe la TRAP totale (TRAP 5a et TRAP 5b); et (b) l'obtention d'une mesure de la quantité de TRAP 5b dans l'échantillon en analysant l'activité de la TRAP 5b liée à l'anticorps à l'aide d'un ester monophosphate d'un composé de phtaléine en tant que substrat, ledit composé de phtaléine étant un composé ayant la formule :

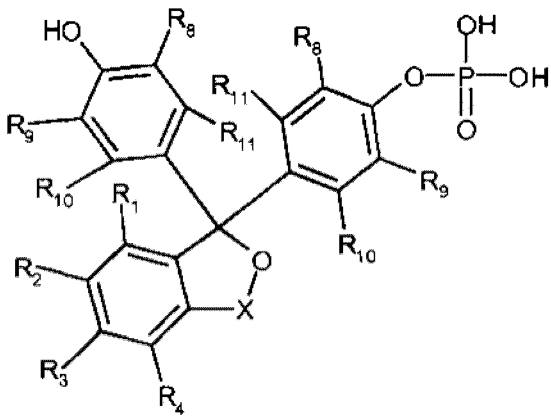

(II)

dans laquelle :

X représente un groupe CO ou $SO_2$;
$R^1$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ et $R^7$;
$R^2$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ et $R^7$;
$R^3$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ et $R^7$;
$R^4$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ et $R^7$;
chaque groupe $R^8$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$,$-C(O)N(R^5)R^6$ et $R^7$;
chaque groupe $R^9$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$,$-C(O)N(R^5)R^6$ et $R^7$;
chaque groupe $R^{10}$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$,$-C(O)N(R^5)R^6$ et $R^7$; et
chaque groupe $R^{11}$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$,$-C(O)N(R^5)R^6$ et $R^7$;

dans lesquels :

n est égal à 0, 1 ou 2 ;
$R^5$ et $R^6$ représentent chacun indépendamment un atome d'hydrogène ou un groupe $R^7$; et
$R^7$ est choisi parmi un groupe hydrocarbyle, $-(CH_2)_k$-hétérocyclyle, $-(CH_2)_k-C(O)$-hétérocyclyle, l'un quelconque desquels étant éventuellement substitué par 1, 2, 3, 4 ou 5 substituants indépendamment choisis parmi un atome d'halogène, un groupe cyano, amino, hydroxy, -COOH et des groupes esters de ceux-ci (par ex. -C(O)-($C_1$ à $C_6$)alkyle), un groupe phosphoramidite, alkyle en $C_1$ à $C_6$ et alcoxy en $C_1$ à $C_6$; et k est égal à 0, 1, 2, 3, 4, 5 ou 6.

**8.** Méthode selon l'une quelconque des revendications 2 à 7 ; ladite étape d'analyse de l'activité de la TRAP 5b étant effectuée à un pH de 6,8 à 7,6, de préférence un pH de 6,8 à 7,4 et idéalement un pH de 7.

**9.** Méthode selon l'une quelconque des revendications 2 à 8, ladite mesure de la quantité de TRAP 5b étant obtenue sous la forme d'une mesure de la couleur de l'échantillon après l'activité de la TRAP 5b.

**10.** Méthode selon l'une quelconque des revendications 1 à 9, ladite étape d'analyse de l'activité de la TRAP 5b dans un échantillon comprenant les étapes séquentielles de :

(a) incubation de l'échantillon avec un ester monophosphate d'un composé de phtaléine pour permettre à la TRAP 5b de métaboliser le substrat;
(b) arrêt du métabolisme;
(c) ajustement éventuel du pH de l'échantillon ;

(d) mesure d'un changement d'une caractéristique du substrat résultant du métabolisme par la TRAP 5b.

**11.** Méthode selon la revendication 9 ou 10, ladite couleur de l'échantillon étant mesurée au moyen d'un spectrophotomètre.

**12.** Méthode selon l'une quelconque des revendications 2 à 10, comprenant en outre l'obtention d'une valeur absolue de la quantité de TRAP 5b présente dans l'échantillon, en comparant la mesure de la TRAP 5b obtenue selon l'une quelconque des revendications à un étalon ou un tableau d'étalonnage.

**13.** Méthode selon l'une quelconque des revendications 2 à 11, ladite étape d'analyse de l'activité de la TRAP 5b étant effectuée sur un substrat solide, de préférence une plateforme, ou un réseau ou une plaque multi-puits.

**14.** Méthode selon la revendication 13, ladite plateforme étant automatisée.

**15.** Méthode selon la revendication 13 ou 14, ladite plateforme automatisée comprenant des billes magnétiques.

**16.** Kit permettant d'exécuter une méthode selon l'une quelconque des revendications précédentes, comprenant un ester monophosphate d'un composé de phtaléine, et un anticorps qui fixe la TRAP totale, ledit composé de phtaléine étant un composé ayant la formule :

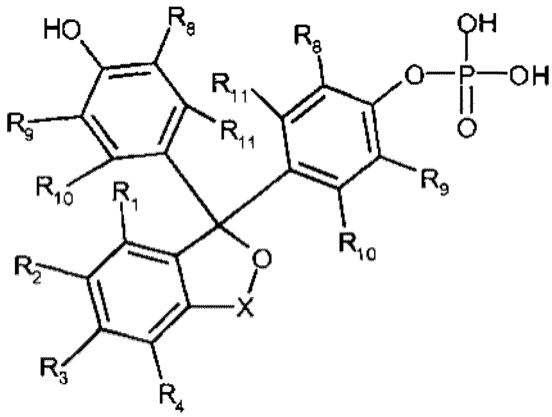

(II)

dans laquelle :

X représente un groupe CO ou $SO_2$;
$R^1$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ et $R^7$;
$R^2$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ et $R^7$;
$R^3$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ et $R^7$;
$R^4$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ et $R^7$;
chaque groupe $R^8$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$, $- C(O)N(R^5)R^6$ et $R^7$;
chaque groupe $R^9$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$, $- C(O)N(R^5)R^6$ et $R^7$;
chaque groupe $R^{10}$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$,$-C(O)N(R^5)R^6$ et $R^7$; et
chaque groupe $R^{11}$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$,$-C(O)N(R^5)R^6$ et $R^7$;

dans lesquels :

n est égal à 0, 1 ou 2;
$R^5$ et $R^6$ représentent chacun indépendamment un atome d'hydrogène ou un groupe $R^7$; et
$R^7$ est choisi parmi un groupe hydrocarbyle, $-(CH_2)_k$-hétérocyclyle, $-(CH_2)_k$-C(O)-hétérocyclyle, l'un quelconque desquels étant éventuellement substitué par 1, 2, 3, 4 ou 5 substituants indépendamment choisis parmi un atome d'halogène, un groupe cyano, amino, hydroxy, -COOH et des groupes esters de ceux-ci (par ex. -C(O)-($C_1$ à $C_6$)alkyle), un groupe phosphoramidite, alkyle en $C_1$ à $C_6$ et alcoxy en $C_1$ à $C_6$; et k est égal à 0, 1, 2, 3, 4, 5 ou 6.

**17.** Utilisation d'un ester monophosphate d'un composé de phtaléine en tant qu'indicateur de la résorption osseuse en agissant comme un substrat spécifique pour la TRAP 5b dans un échantillon, ledit composé de phtaléine étant un composé ayant la formule :

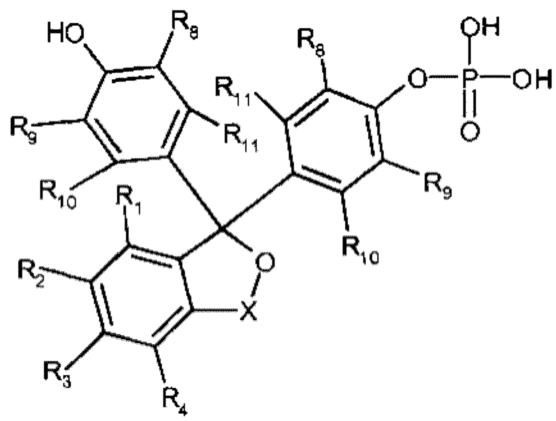

**(II)**

dans laquelle :

X représente un groupe CO ou $SO_2$;
$R^1$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ et $R^7$;
$R^2$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ et $R^7$;
$R^3$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ et $R^7$;
$R^4$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$, $-C(O)N(R^5)R^6$ et $R^7$;
chaque groupe $R^8$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$,$-C(O)N(R^5)R^6$ et $R^7$;
chaque groupe $R^9$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$,$-C(O)N(R^5)R^6$ et $R^7$;
chaque groupe $R^{10}$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$,$-C(O)N(R^5)R^6$ et $R^7$; et
chaque groupe $R^{11}$ est choisi parmi un atome d'hydrogène, d'halogène, un groupe trifluorométhyle, cyano, nitro, $-OR^5$, $-C(O)R^5$, $-C(O)OR^5$, $-OC(O)R^5$, $-S(O)_nR^5$, $-N(R^5)R^6$,$-C(O)N(R^5)R^6$ et $R^7$;

dans lesquels :

n est égal à 0, 1 ou 2 ;
$R^5$ et $R^6$ représentent chacun indépendamment un atome d'hydrogène ou un groupe $R^7$; et
$R^7$ est choisi parmi un groupe hydrocarbyle, $-(CH_2)_k$-hétérocyclyle, $-(CH_2)_k$-C(O)-hétérocyclyle, l'un quelconque desquels étant éventuellement substitué par 1, 2, 3, 4 ou 5 substituants indépendamment choisis parmi un atome d'halogène, un groupe cyano, amino, hydroxy, -COOH et des groupes esters de ceux-ci (par ex. -C(O)-($C_1$ à $C_6$)alkyle), un groupe phosphoramidite, alkyle en $C_1$ à $C_6$ et alcoxy en $C_1$ à $C_6$; et k est égal à 0, 1, 2, 3, 4, 5 ou 6.

**18.** Utilisation selon la revendication 17 dans une méthode selon l'une quelconque des revendications 1 à 15.

**19.** Utilisation selon la revendication 17, comprenant la mise en contact de l'ester monophosphate avec un échantillon provenant d'un sujet et la mesure ultérieure de la couleur de l'échantillon.

**20.** Utilisation, méthode ou kit selon l'une quelconque des revendications précédentes, ledit composé de phtaléine étant une phénolphtaléine ou une sulfophtaléine.

**21.** Utilisation, méthode ou kit selon la revendication 20, ledit composé de phtaléine étant une phénolsulfophtaléine.

**22.** Utilisation, méthode ou kit selon l'une quelconques des revendications précédentes, ledit ester monophosphate d'un composé de phtaléine étant choisi dans le groupe constitué par le monophosphate de phénolphtaléine, le monophosphate de crésolphtaléine, le monophosphate de thymolphtaléine, le monophosphate de o-chlorophénolphtaléine, le monophosphate de rouge de phénol, le monophosphate de rouge de o-crésol, le monophosphate de violet de m-crésol, le monophosphate de violet de bromocrésol, le monophosphate de vert de bromocrésol, le monophosphate de bleu de bromophénol, le monophosphate de bleu de thymol, le monophosphate de bleu de bromothymol, le monophosphate de bleu de xylénol et le monophosphate de rouge de chlorophénol.

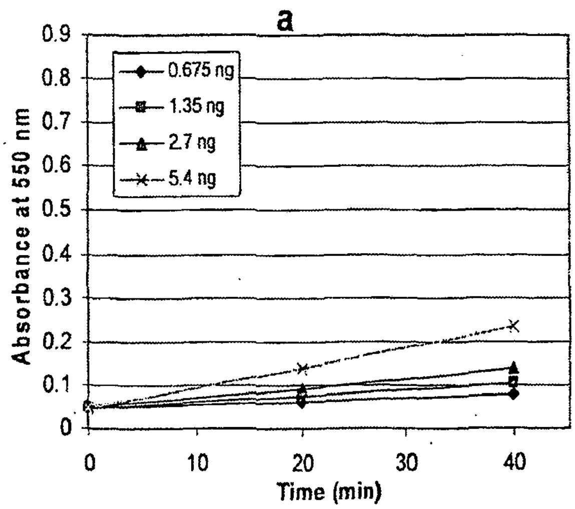


Figure 1a

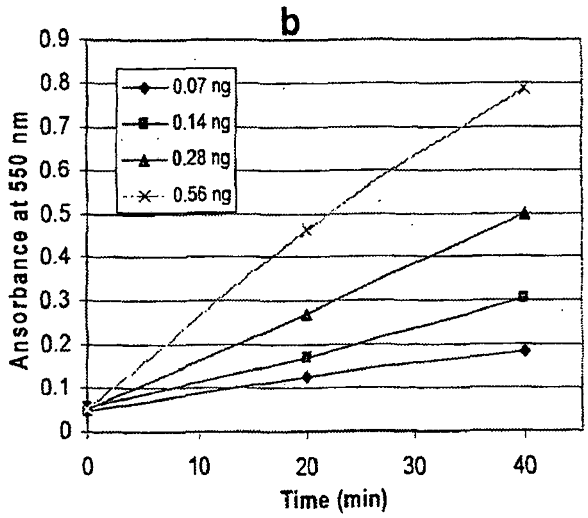


Figure 1b

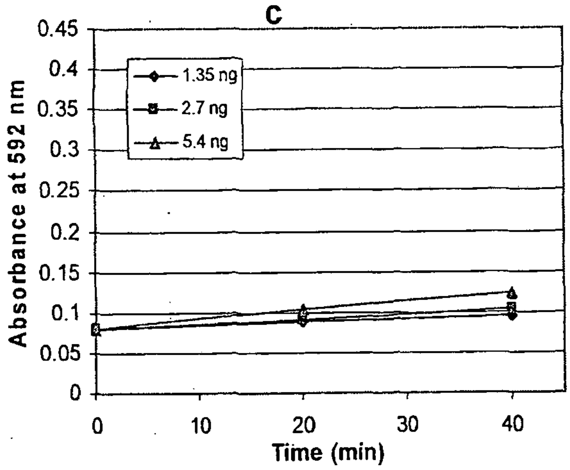


Figure 1c

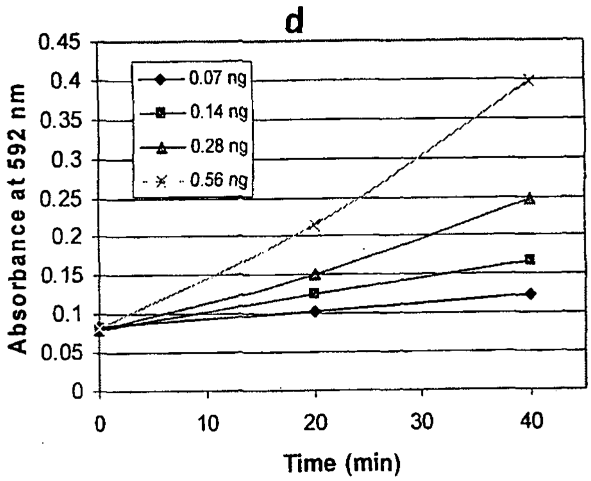


Figure 1d

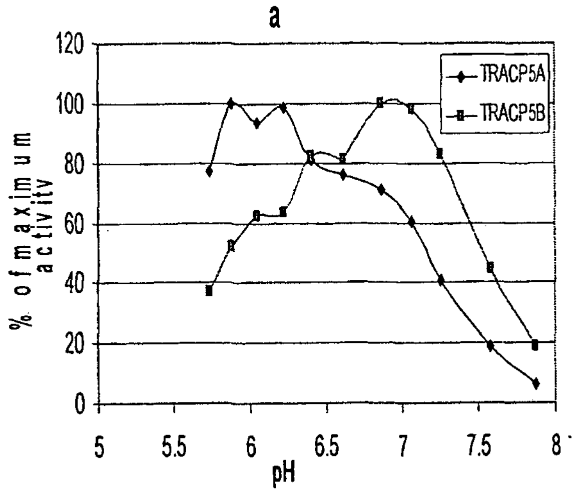
a
120
100
80
60
40
20
0
% of maximum activity
TRACP5A
TRACP5B
5
5.5
6
6.5
7
7.5
8
pH

Figure 2

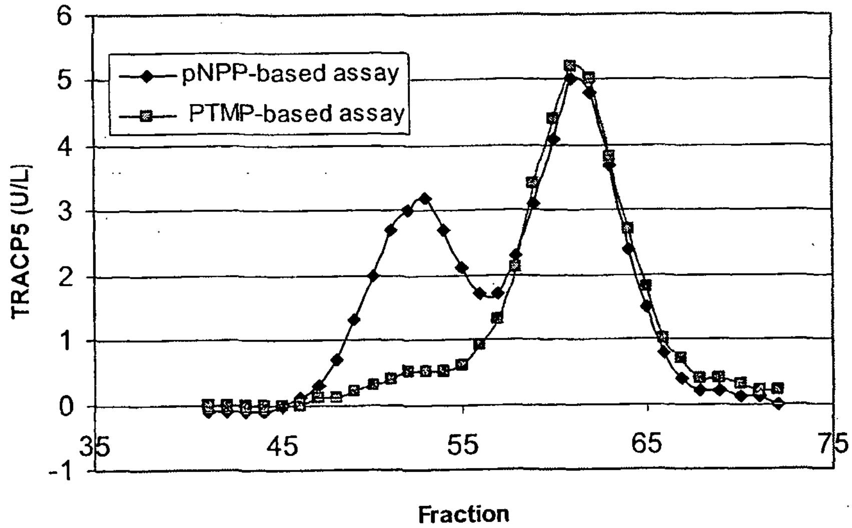
6
5
4
3
2
1
0
-1
TRACP5 (U/L)
pNPP-based assay
PTMP-based assay
35
45
55
65
75
Fraction

Figure 3

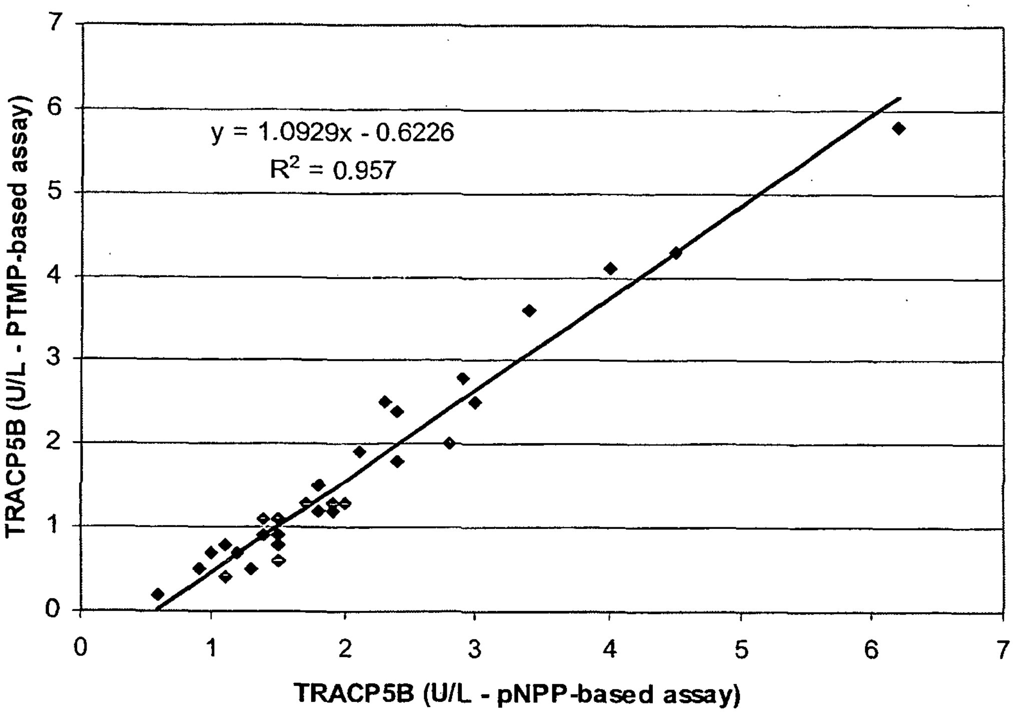
y = 1.0929x - 0.6226
R2 = 0.957
TRACP5B (U/L - PTMP-based assay)
TRACP5B (U/L - pNPP-based assay)
0
1
2
3
4
5
6
7

**Figure 4**

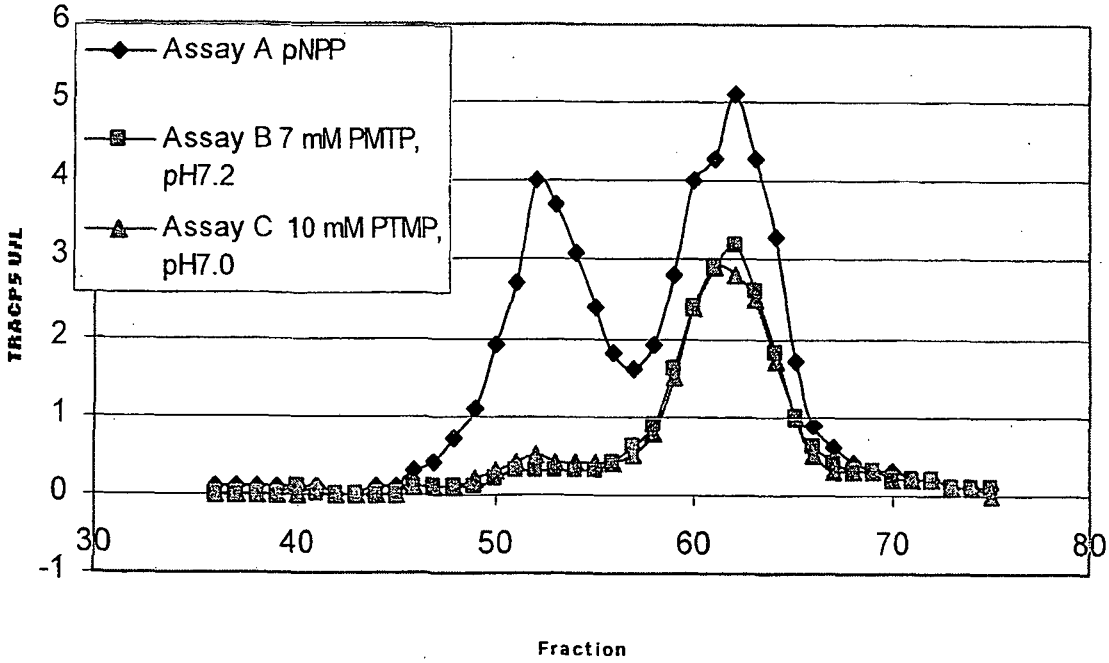
Assay A pNPP
Assay B 7 mM PMTP, pH7.2
Assay C 10 mM PTMP, pH7.0
TRACP5 U/L
6
5
4
3
2
1
0
-1
30
40
50
60
70
80
Fraction

Figure 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 1999050662 A
- EP 1598670 A
- US 7074903 B
- EP 1359161 A
- US 3975405 A
- US 4045290 A


### Non-patent literature cited in the description


- **HAYMAN, A. R et al.** *Development,* 1996, vol. 122, 3151-3162
- **KRAENZLIN M. E et al.** *Journal of Clinical Endocrinology and Metabolism,* 1990, vol. 71 (2), 442-451
- **CHEUNG. C.K et al.** *Clin. Chem,* 1995, vol. 41 (5), 679-686
- **CHAMBERLAIN P. et al.** *Clin. Chem.,* 1995, vol. 41 (10), 1495-1499
- **HALLEEN J. et al.** *Journal of Bone and Mineral Research,* 1996, vol. 11 (10), 1444-1452
- **LAM W. K. W. et al.** *Clin. Chem.,* 1978, vol. 24 (7), 1105-1108
- **STEPAN J.J et al.** *Biochem, Biophs. Res. Commun.,* 1989, vol. 165, 1027-1034
- **KRAENZLIN M.E et al.** *Journal of Clinical Endrocrinology and Metabolism,* 1990, vol. 71 (2), 442-451
- **CHEUNG C. K et al.** *Clin. Chem.,* 1995, vol. 41 (5), 679-686
- **HALLEEN J. et al.** *Journal of Bone and Mineral Research 11,* 1996, vol. 10, 1444-1452
- **CHAMBERLAIN P.** *Clin. Chem.,* 1995, vol. 41 (10), 1495-1499
- **HALLEEN et al.** *JBMR,* 2000, vol. 15, 1335-1434
- Practice and Theory of Enzyme Immunoassay. Laboratory Techniques in Biochemistry and Molecular Biology. vol. 15
- **HALLEEN et al.** *JMBR 14,* 1999, 464-9
- **JANCKILLA et al.** *Hybridoma,* 1997, vol. 16, 175-82
- **RMC DAWSON et al.** Data for Biochemical Research. Clarendon Press, 1989
- **LAM et al.** *Clin. Chem.,* 1978, vol. 24 (7), 1105-1108